# EUROPEAN PATENT SPECIFICATION

(11) **EP 3 495 038 B1**
(45) Date of publication and mention of the grant of the patent: **10.06.2020**
(21) Application number: 17206537.7
(22) Date of filing: 11.12.2017
(51) Int. Cl.: B01J 19/00, B04B 5/04

(54) **DEVICE FOR PARALLEL OLIGOMER SYNTHESIS, METHOD OF PARALLEL OLIGOMER SYNTHESIS AND USE THEREOF**
VORRICHTUNG ZUR PARALLELEN OLIGOMERSYNTHESE, VERFAHREN ZUR PARALLELEN OLIGOMERSYNTHESE UND VERWENDUNG DAVON
DISPOSITIF DE SYNTHÈSE D'OLIGOMÈRES PARALLÈLES, PROCÉDÉ DE SYNTHÈSE D'OLIGOMÈRES PARALLÈLES ET SON UTILISATION

(43) Date of publication of application: 12.06.2019
(73) Proprietor: The Institute of Organic Chemistry and Biochemistry AV CR, 16610 Prague (CZ); Spyder Institute Praha s.r.o., Dolni Jircany 25244 Psary (CZ)
(72) Inventor: Lebl, Michal, 11800 Prague (CZ); Flegelova, Zuzana, 25244 Psary (CZ); Poncar, Pavel, 14100 Prague (CZ); Mudra, Petr, 19600 Prague (CZ); Paces, Ondrej, 150 00 Prague (CZ); Knor, Matyas, 26718 Hlasna Treban (CZ); Buzek, Michal, 16300 Prague (CZ); Smrz, Jiri, 109 00 Prague (CZ); Pokorny, Vit, 15200 Prague (CZ); Pesek, Vaclav, 14100 Prague (CZ)
(74) Representative: Hartvichova, Katerina

(56) References cited:
- WO-A1-01/54815
- US-A1- 2007 110 637
- US-A1- 2013 252 796
- MICHAL LEBL ET AL: "Economical Parallel Oligonucleotide and Peptide Synthesizer - Pet Oligator", INTERNATIONAL JOURNAL OF PEPTIDE RESEARCH AND THERAPEUTICS ; FORMERLY KNOWN AS LETTERS IN PEPTIDE SCIENCE, KLUWER ACADEMIC PUBLISHERS, DO, vol. 13, no. 1-2, 2 May 2007 (2007-05-02), pages 367-375, XP019505776, ISSN: 1573-3904, DOI: 10.1007/S10989-007-9096-X

## Description

### Field of Art

The present invention relates to a device for parallel oligomer synthesis based on parallel centrifugal processing of reactors, a method of parallel oligomer synthesis and use thereof.

### Background Art

Oligomer (peptide, oligonucleotide, carbohydrate) synthesis is based on the repetition of similar organic reactions resulting in connecting building units (amino acids, nucleotides, sugars) to form macromolecular organic compounds (peptides, oligonucleotides, carbohydrates). There are various automated synthesizers designed for synthesis of these oligomers based on multitude of technologies. Examples of such automated synthesizers are briefly discussed below.

US 2007/110637 A1 describes an automated rotary synthesizer comprising a plurality of reaction holders retaining reactors at an angle, and a dosing device. The reaction wells are moved by a carousel into allignment with stationary nozzles, communicating with the source of reactants or washing solutions.

US 2013/252796 A1 discloses a device for insertion into a rotor of a centrifuge, containing two bodies having a cavity. The bodies are movable disposed in relation to each other in order to fluidically couple in response to the rotation of the centrifuge.

WO 01/54815 A1 describes a centrifugal dispenser, supplying fluid to a dispensing element on a centrifugal rotor by centrifugal source.

The system described for instance in the US patent No. 8,731,721 or 7,914,739 is capable of continuously synthesizing molecules by providing an array of reaction sites and an array of stations for carrying out synthetic manipulations. The reaction sites and the stations are placed such that the two arrays can be moved relative to each other such that the stations carry out desired steps of a reaction scheme at each reaction site.

The system contains a rotary table with plate modules located along the outer circumference of the table. The plate modules contain plate holders, which support microtiter plates. During operation, the microtiter plates pass under dispensing stations, such that the movement of the table and placement of the stations allows all microtiter plates to receive reactants and incubate the reactants according the predetermined reaction scheme, wherein each well of the microtiter plate is assigned a different oligonucleotide to be synthesized based on predetermined requirements. Based on this assignment, the program instructs each dispenser to output the proper reaction solutions into the proper well.

However, there are many laboratories synthesizing these compounds manually, requiring performance of repetitive processes - addition of reagents, incubation and separation of liquid-phases, which are prone to the human errors resulting from momentary attention lapses. One of the popular techniques for oligomer synthesis utilizes simple disposable plastic syringes. Major advantage of this technique pioneered by Krchnak et al. (Tetrahedron Letters, Volume 28, Issue 38, 1987, Pages 4469-4472, Continuous-flow solid-phase peptide synthesis, Viktor Krchňák, Josef Vagner, Martin Flegel, Otakar Mach, http://dx.doi.org/10.1016/S0040-4039(00)96541-9) is the fact that the syringe is capable of handling majority of organic reagents and can be used under various conditions in enclosed environment. This allows combination of the routine building of the oligomer with very sensitive reactions requiring special attention and conditions.

For instance, the US patent No. 8,394,923 discloses a centrifugation based apparatus and method for separating layers of immiscible or partially miscible liquids of different density by removal of lighter layers of liquids by creation of "pockets" from which material cannot be removed by centrifugal force.

In this way, the liquid phase can be eliminated from reaction vessels, where the synthesis occurs. The reaction vessels are held in a tilted position with a tilt towards the axis of rotation. The rotor is then spun at a speed that expels the liquid from the vessels, keeping at the same time the solid support in "pockets" created by centrifugation.

Further, a US patent No. 8,022,013 discloses a method of synthesis, wherein a solid-phase support is provided, for instance macrobeads or microbeads, a particle is dispensed in the reaction vessel, permanently bonded in the substrate within the reaction vessel, and functionalized to covalently attach an intermediate compound of a synthetic reaction, a liquid including a reagent is dispensed to the solid-phase support to effect the synthetic reaction, and the liquid is removed from the solid-phase support by centrifugation, whereby the intermediate compound remains attached to the substrate by the particle. The substrate and support are capable of withstanding centrifugal forces generated during high-throughput synthesis.

The solid-phase beads may be held within the vessel during centrifugation by tilting the reaction vessel to form pockets from which the solid-phase cannot be removed by centrifugal force, as described above. Alternatively, mesh or other frit materials can be employed.

Michal Lebl et al., International Journal of Peptide Research and Therapeutics, vol. 13, no. 1-2 (2007) p. 367-375, discloses a device for parallel oligonucleotide synthesis comprising a centrifuge with a plurality of reaction vessels and a distribution system above it. Centrifugation, which is used in the device instead of filtration, allows for independent individual wells in the solvent removal operation.

All described technologies are limited in the scale applicable to the synthesis and relatively complicated hardware. Tilted centrifugation is good for the synthesis using non-swelling carrier forming the same volume of the solid support throughout the whole synthesis. This type of carrier is suitable for synthesis of oligonucleotides, but it is not usable for peptide synthesis where the carrier can grow substantially during the synthesis. All methods using mesh or frit material to retain solid support must solve a problem of leakage of the liquid through the supporting material during incubation periods.

The first set of available background art documents constitute automatized arrays for performing syntheses as described above. In all of the above described methods of oligomer synthesis, a human intervention is needed. For example, in the methods using centrifugation, each removal of a reagent solution from the reaction vessel after centrifugation is done manually, which incorporates potential errors into the oligomer synthesis, and causes delays, which slow down the synthetical process. Another disadvantage lies in the fact that not complete removal of the liquid from the centrifugation tube after centrifugation can be performed by aspiration of the liquid. There is still certain amount of liquid present in the solid sample even after its careful aspiration, which introduces potential errors into the oligomer synthesis.

### Disclosure of the invention

The presented invention allows the operator to run long oligomer (peptide, oligonucleotide, carbohydrate) synthesis in parallel automatically, i.e. without human interaction, therefore overcoming the problems of the background art. In any step of the synthesis, however, the operator may remove an individual reactor and perform a special operation requiring special conditions or utilizing very sensitive or special reactant manually. After that step, the reactor can be returned to the device for continuation of the oligomer assembly. Such procedure enables the operator to parallelly synthesize a number of oligomers either in a fully automatic way, or, upon his choice, to interrupt the automated synthetical procedure at any stage in order to perform one or several consecutive synthetical steps manually, and then to return back to the automated synthetical procedure. The device according to the present invention therefore enables the synthesis of oligomers containing even very sensitive, costly or valuable monomeric units, which need special treatment and conditions. Moreover, the S-trap outflows present in the device according to the present invention (as described below) allow for a complete removal of liquids during centrifugation, which overcomes the other above discussed disadvantages of the background art.

The concept of the liquid reactant delivery for the parallel oligomer synthesis is based on parallel centrifugal processing of reactors. A device for parallel oligomer synthesis comprises the following subsystems: centrifugation and outflow subsystem and dosing subsystem, which can be supplied by selection subsystem and storage. All of them may be retained by a boxing subsystem. They are controlled by a control subsystem.

The synthesis takes place in reactors. Each of the reactors has an upper end and a lower end. The upper end is an opened end and the lower end comprises a nozzle. The reactors may be constituted by syringes, preferably made of plastic. However, reactors may be made of any other suitable material in any suitable form in order to stay resistant against the solvents used during the synthesis. A person skilled in the art would be able to decide, which material should/should not be contacted with which solvent.
Inside each reactor, a filter may be provided at the lower end close to (above) the nozzle to provide a semipermeable membrane. As such, it provides a stopper for the solid phase (e.g. resin beads provided with functional groups suitable for immobilization of amino acids used for peptide synthesis or building blocks used in the synthesis of other biooligomers (nucleic acids, carbohydrates)), which is placed in the reactors at the beginning of the synthesis, while it allows the reagents and/or solvents to be washed out of the reactor after completion of each step of the oligomer synthesis. The filter suitable for being placed inside the reactor is, for instance, a sintered glass, plastic (polypropylene, Teflon) or metal mesh.

The nozzle of each reactor is connected to an S-trap outflow. The S-trap outflow is a tube in S-shape and comprises a first end and a second end. The first end of the S-trap outflow is connected to the nozzle of the lower end of the reactor. The S-trap outflow provides an outflow for reagents to be removed during the synthesis and works on a siphoning principle, i.e. the second end of the S-trap outflow, when placed in the device, is positioned higher than the level of contents of the reactor. Thus, depending on the speed of rotation, the liquid in reactor is either not capable to overcome the gravity based resistance of the siphon at low speed, or it is removed completely from the reactor at high speed rotation by centrifugal forces. The low speed of rotation can be defined by up to 50 rpm, more preferably up to 40 rpm, more preferably from 5 to 30 rpm, even more preferably from 10 to 20 rpm, most preferably about 10 rpm. The medium speed of rotation can be defined by an interval from 50 to 200 rpm, more preferably from 80 to 150 rpm, more preferably from 100 to 130 rpm, most preferably about 120 rpm. The high speed of rotation can be defined by more than 200 rpm, more preferably from 250 to 6000 rpm, more preferably from 500 to 3000 rpm, even more preferably from 800 to 2000 rpm, most preferably about 1000 rpm.

The second end of the S-trap outflow may also comprise a nozzle. The second end of the S-trap outflow or the nozzle of the S-trap outflow is positioned so that liquids removed from the reactor through the second end of the S-trap outflow or the nozzle of the S-trap outflow flow into a circular outflow channel, which will be described in more details later.

The reactors and the S-trap outflows are positioned in a centrifugation subsystem comprising a centrifuge. The centrifuge is a circular disk, made of inert material, preferably metal, for example steel, aluminum, solvent resistant plastic, glass, etc. and seated on a center axle.

The centrifuge is driven by a driving motor seated on the same common axle as the centrifuge. The driving motor allows the centrifuge to rotate at different speeds, from low (of about 10 rpm), through medium (of about 50 to 200 rpm), to high (of about 1000 rpm) speed of rotation. It also allows a stepped motion.

The centrifuge comprises reactor holders, which are configured to retain the reactors tilted towards the vertical axle of the centrifuge at an angle. The reactors may be positioned at an angle of 30 to 85 degrees, preferentially at the angle of 45 degrees. The holders may be constituted by fixation slots, in which at least parts of the reactors are fixed, such that the reactors are securely fixed during the synthesis, but freely removable when the centrifuge is stopped.

In one embodiment, the centrifuge may comprise two circular disks - an upper disk and a lower disk, both of the disks being made of an inert material and seated on a common axle in the center of mass. Vertically, the upper and the lower disks may be positioned at a distance from each other. The distance between the upper and the lower disk is determined by the size of the reactors. Even though the disks are positioned at a distance, they are physically connected (e.g. with use of screws and supporting brackets) to each other. The first and the second disk may be manufactured as a one construct combining the function of disks and S-trap outflows by e.g. additive manufacturing.
In the above-mentioned embodiment, the upper disk is provided with reactor holder openings at its inner circumference and with S-trap outflow openings at its outer circumference. The lower disk is provided with fixation slots for the nozzles of the reactors and with the S-trap outflow openings in the fixation slots. The fixation slots with S-trap outflow openings are provided at the lower disk's outer circumference. The reactor may then be inserted through the opening in the upper disk into the fixation slot of the lower disk. As the opening is provided at the inner circumference and the fixation slot is provided at the outer circumference, the reactor is positioned at an angle with respect to the central vertical axle of the centrifuge, e.g. at an angle of 45 degrees. The reactors are securely fixed during centrifugation, but they can be easily removed from reactor holders when the centrifuge is static. The reactors can be removed by hand.

The reactors and the S-trap outflows are positioned such that the content of liquids in the reactor reaches a level which is vertically lower than the second end of the S-trap outflow. The S-trap outflow thus operates on a siphoning principle: when the rotation speed of the centrifuge is low, the liquid in the reactor is not capable of overcoming the gravity based resistance of the S-trap outflow and remains fully inside the reactor. When the rotation speed of the centrifuge increases, the liquid from the reactor enters the S-trap outflow from the nozzle of the lower end of the reactor and continues through the S-trap outflow, and, depending on the speed of rotation, the liquid may be removed from the reactor through the S-trap outflow partially or completely. This enables the oligomer synthesis to run in parallel and in full cycle, including several partial or complete washing cycles. Thus, removal of liquids using centrifugation and S-trap outflow is significantly more efficient than a simple aspiration of liquids. The rotation speed of the centrifuge required for the liquid from the reactor to overcome the S-trap may partially depend on the solvent, particularly on the density and viscosity of the solvent. A person skilled in the art would be able to decide, which speed of the centrifuge should be used with a particular solvent.

The liquids are removed from the S-trap outflow through the second end or the nozzle of the S-trap outflow and flow into the circular outflow channel, which is positioned on the perimeter of the centrifuge and aligned with the second ends or the nozzles of S-trap outflows such that the second ends or the nozzles of the S-trap outflows are positioned in the circular outflow channel. The circular outflow channel comprises outflow openings positioned on its bottom. The outflow openings of the outflow channel are connected to outflow pipes, which provide an outflow system of the device and drain away the liquid removed from the reactors during the synthesis.

Above the centrifugation disk, a distribution rotor is positioned. It has a shape of a circular disk and is made of a suitable inert material, e.g. of the same material as the centrifuge. The distribution rotor and the centrifuge have the same common axis, however, the distribution rotor is seated on its own axle and it is connected to a positioning device, which comprises a driving motor to allow positioning of the distribution rotor independently from the centrifuge. The motor driving the distribution rotor allows the distribution rotor to rotate at different speeds and in a stepped motion. The independent movement of the distribution rotor, in particular the stepped motion, allows each of the individual pre-activation compartments (will be described later) to be filled in separately, one by one in an automated way.
The distribution rotor is further movable in vertical direction between a lower position and an upper position. The vertical movement is provided by a lifting device, which may be e.g. another motor or a gas operated piston. The vertical movement is provided in order to allow the distribution rotor to be in contact with the centrifuge in the lower position to ensure a synchronized rotation of the centrifuge and the distribution rotor.
Both the positioning device and the lifting device are positioned above the distribution rotor, outside of a centrifugation drum, in which the centrifuge and the distribution rotor are placed. The distribution rotor is driven by its own driving motor only when it is positioned in the upper position. In the upper position, it is detached from the centrifuge, which can then perform independent operations with the reactors (shaking, rotation, centrifugation). When the distribution rotor is positioned in the lower position, the motor driving the distribution rotor is disengaged from the distribution rotor and all actuation of the distribution rotor is performed by the motor driving the centrifuge so that the synchronized rotation of the centrifuge and the distribution rotor is ensured.

The distribution rotor comprises pre-activation compartments. Reagents are delivered into the pre-activation compartments such that the pre-activation of non-activated building blocks can be achieved. The reagents may be delivered into the pre-activation compartments by a dosing subsystem, which will be described later. The pre-activation compartments are positioned at the outer circumference of the distribution rotor and are provided with grooves directed radially outwards, towards the outer edge of the distribution rotor. At the stage of no rotation or a slow rotation (of about 10 rpm), the liquids contained in the pre-activation compartments cannot overcome the tilt of the grooves, and therefore stay in the pre-activation compartments. These grooves enable the connection between the pre-activation compartments of the distribution rotor and centrifuge when the distribution rotor is in the lower position such that the contents of the compartments may be transferred to the reactors upon synchronized rotation of the distribution rotor and the centrifuge at medium rotation speed (from ca 50 to ca 200 rpm). At the medium speed, the liquids are transferred from each of the pre-activation compartments into the corresponding reactor through the corresponding groove, however, they remain inside the reactor because the speed of rotation is not high enough to overcome the resistance of the S-trap outflow, and the liquids are therefore prevented from flowing out of the reactor.
As the rotation of the centrifuge and the distribution rotor is synchronized, this transfer may be provided for all pre-activation compartments of the distribution rotor and the corresponding reactors in parallel.

The pre-activation compartments can be filled in directly or via a dosing device.
In one embodiment, the dosing device is enabled in the system. The dosing device may comprise a distribution valve and a dosing pump, the dosing pump being connected to the distribution valve. The dosing pump may be configured to measure the exact quantity of liquid reagents to be dosed into individual pre-activation compartments. The dosing pump may be a programmable syringe pump.

The distribution valve comprises a plurality of ports. The distribution valve comprises at least one outtake port which is via tubing connected to the centrifugation subsystem and through which the individual pre-activation compartments may be filled in. The other ports of the distribution valve are intake ports and provide for a connection with stock solutions of reactants and/or solvents and/or activating reagents and/or coupling reagents stored in storage containers.

The dosing pump may be connected to the desired reactant via one of the ports of the distribution valve (intake connection; the other ports are closed) and a desired quantity may be sucked by the dosing pump inside the dosing pump. Subsequently, the intake connection may be closed and an outtake connection between the dosing pump and the distribution rotor may be opened.

In one embodiment, a distribution device may be enabled in the system. It connects the dosing pump via the distribution valve to either the selector valve or to the pre-activation compartments of the distribution rotor. The distribution device may be a solenoid valve with three ports switching between the connection of the dosing pump with the outtake port of selector and the connection between the dosing pump and the pre-activation compartments of the distribution rotor (through the distribution valve).

Depending on whether the distribution device is or is not enabled in the system, the distribution valve may comprise up to 12 ports. For example, the distribution valve may have 10 ports when there is no distribution device enabled in the system and 12 ports when there is the distribution device enabled in the system. Alternatively, a multiport rotary valve may be enabled in the system instead of the three-way solenoid valve.

In one embodiment, it is possible to place a tubing with a predefined volume (which is larger than the volume being delivered to the reactor) between the dosing pump and inlet opening of the pre-activation compartment to avoid the reagents entering the dosing pump inner volume. In this arrangement, the distribution device must be placed between selector valve and inlet port of the centrifuge assembly. The distribution device (e.g. the three-way solenoid) is opened in the direction to the dosing pump, liquid is sucked from the distribution valve into the tubing, solenoid is opened in direction of delivery to the particular reactor and liquid is expelled into the reactor, without entering the syringe of the dosing pump and contaminating the liquid in there.

In any case, the desired quantity of the reactant is transferred via tubing and one of the pre-activation compartments is filled in. This process is repeated with either the same reactant or other reactants until the desired number of pre-activation compartments is filled in.

The building blocks, for example amino-acids, for the oligomer synthesis may be stored in storage containers. The storage containers may either be connected directly to the distribution valve of the dosing device or they may be connected through a selector device.

In one embodiment, one of the intake ports of the distribution valve may be used for connection to a selection device. The selection device is a valve configured to select the desired building block solution (e.g. solution of a particular amino-acid in oligopeptide synthesis), which may then be transferred through the selector device, the distribution valve and the dosing pump into the individual pre-activation compartments of the distribution rotor.

The selector device comprises a plurality of ports. Preferably, it may comprise up to 28 intake ports. The intake ports are connected via tubing with the storage containers containing various building block solutions (e.g. amino-acid solutions).

The selector device further comprises an outtake port, which may be connected via tubing with one of the intake ports of the distribution valve of the dosing device. In order to retrieve the desired amino-acid, the connection between the dosing pump, one of the intake ports of the distribution valve, the outtake port of the selector device and one of the intake ports of the selector device is opened and the desired quantity of the desired amino-acid is sucked from the storage container into the dosing pump. The desired quantity of the desired amino-acid is then transferred from the dosing pump through the outtake port of the distribution valve into one of the pre-activation compartments.

In one embodiment, one of the output ports of the dosing pump may be selected to be used exclusively for washing, i.e. for providing a connection to washing solvent only, as it is likely that larger volumes of washing solvents are used during the entire synthesis, or, alternatively, washing solvents can be delivered by separate dosing pump.

In one embodiment, the selector device may comprise coplanar layers of graphite pressed together by spring. Two graphite layers touching each other have extraordinary sealing properties, while at the same time having a very low friction coefficient. Using of graphite for the selector device is especially advantageous as there is no need for further sealing additives or friction reducing additives. The selector device may comprise two coplanar, preferably circular, graphite layers. The upper layer comprises a plurality of holes, or the intake ports, respectively. Preferably, the intake ports are located close to the outer circumference of the upper graphite layer. The lower graphite layer comprises a single hole, or the outtake port, respectively, and an elongated groove. The outtake port is located in the middle of the circular graphite layer. The elongated groove has a first end and a second end. The position of the first end of the groove is identical with the position of the outtake port. The groove is elongated radially outwards form the position of the first end of the groove such that the position of the second end of the elongated groove is identical with position of one of the intake ports in the upper graphite layer. Thus, a connection between the intake port, the elongated groove and the outtake port is established. As the intake ports are positioned in a circular configuration along the outer circumference of the upper graphite layer, the elongated groove allows to establish a connection between the outtake port and any one of the intake ports by rotating of one of the two coplanar graphite layers relatively to the other one. In order to ensure the non-crosscontamination of the desired reactant by the reactant from any other unselected port, this coplanar arrangement selector port should preferably be used in underpressure (suction) regimen, when the sealing of the two coplanar surfaces can be guaranteed.

The solutions of particular building blocks (e.g. amino-acids) may be stored in storage containers. The storage containers may have various sizes and may be made of any suitable materials resistant to the solvent used, preferably plastic. The number of storage containers may correspond to the number of intake ports of the selector device. Thus, it may contain up to 28 storage containers. In one embodiment, the storage containers may have unified shape, for example cylindrical shape and may be disposably placed in a holder. The holder may have openings into which the storage containers may be placed. In this way, all the storage containers are securely placed in one holder.

The centrifugation subsystem and the distribution rotor may be placed in a centrifugation drum. The centrifugation drum may have a cylindrical shape with diameter larger than the diameter of the centrifuge, such that the centrifuge fits in the centrifugation drum.

The centrifugation drum may be placed in a box. The box has an upper part with a circular opening in this upper part, into which the centrifugation drum may be placed. Preferably, in order to minimize the space required for the device, the upper part may further serve as a storage place for the rest of the equipment: the dosing device, the selector device and the holder with the storage containers.

Part of the outflow system may also be placed in the box. As was discussed above, the circular outflow channel comprises outflow openings, which are connected to outflow pipes, which provide an outflow system of the device and drain away the liquid removed from the reactors during the synthesis. These outflow pipes may be combined into one outflow tube, which is positioned within the box.

The centrifuge and the distribution rotor may be sealed within the centrifugation drum. The sealing may be provided by a cover of the centrifugation drum.

The cover may be rotatably attached to the upper part of the box and may rotatably move from an open position to a closed position and vice versa. Preferably, the cover is placed between the distribution rotor and the positioning device (and the lifting device), as both of them are placed above the distribution rotor and outside of the centrifugation drum. The cover may be operated manually or automatically by the control subsystem. The positioning device and the lifting device are then operated together with the cover. The sealing shall be activated in the closed position of the cover, such that the environment within the centrifugation drum is insulated from the space outside the centrifugation drum. The cover may be made of an inert material, for example the same material may be used as is used for the manufacture of the centrifuge. Alternatively, the material may be transparent, for instance plexiglass protected by a sheet of inert material or glass. The transparent material has an advantage that the operator may watch the operation of the device directly.
In one embodiment, the cover may comprise two concentric disks - the inner disk and the outer disk. In this embodiment, the inner disk is preferably made of steel, but may be made of any suitable inert material and the outer disk is preferably made of glass, but may also be made of any suitable inert material. To ensure the safety of the operation, it may be provided that the rotation of both rotors can be enabled only when the seal is in place. There is at least one opening - inlet port in the cover, through which the centrifugation subsystem may be connected to the dosing device.

The sealed cover may especially be important when certain conditions need to be provided inside the centrifugation drum. For instance, in order to provide such conditions inside the centrifugation drum, a temperature control device may be provided inside the centrifugation drum in one embodiment. The temperature control device may comprise for example an infrared radiator and a microwave radiator. Such a temperature control device may provide temperatures up to 90 degrees Celsius. The temperature control device may further comprise a sensor with a feedback control and may be controlled automatically by the control subsystem.

In one embodiment, the device may further comprise a first plurality of magnets, preferably at least two, which may be attached statically to the inner side of the centrifugation drum at the level of the reactors. In this embodiment, a second plurality of magnets is further positioned inside the reactors (i.e. preferably one magnet in each of the reactors). The size of the magnets placed inside the reactors is smaller than the size of the magnets attached to the centrifugation drum. Because of the interaction of the magnets inside the reactors with magnets attached to the centrifugation drum, the resin inside the reactors is thoroughly stirred during a slow rotation (from ca 10 rpm to ca 50 rpm) of the centrifuge in the synthetic process. The addition of magnets is especially advantageous for larger quantities of contents inside the reactor or for large reactor sizes suitable for large volumes of reagents, when simple rotation and/or shaking of the centrifuge is not sufficient to stir the contents to a desired extent.

Every part of the system is controlled by the timing protocol of the program specifically constructed for the presented device. The input data comprise a list of oligomers (e.g. peptides) to be synthetized with specific individual sequences of monomer building blocks (e.g. aminoacids). The device according to the present invention is capable of parallel synthesis of a plurality of different oligomers (e.g. oligopeptides with different aminoacid sequences). The timing protocol of the whole process is then based on the input data. The program allows to check spots of difficult coupling and suggests the best protocol, such that every step may have different protocol (different coupling time, reagent volume etc.). The software also allows the operator to interrupt the process at any time and perform the particular step manually, if needed.

Reagents shall be prepared in storage containers, which shall be connected to the device via the dosing device and/or selector device. When the device is prepared, the control device retrieves the sequences of the particular oligomers (e.g. peptides) to be synthesized and determines the sequence specific timing protocol. The principal use of this method is in the peptide synthesis.
The peptide synthesis performed by the above-described device follows the timing protocol and comprises the following steps:
a) resin beads provided with functional groups suitable for immobilization of amino acids are placed in each reactor, eventually equipped with a small magnet placed inside the reactors. The resin beads used for the peptide synthesis are selected from the group comprising polystyrene resins, polyamide resins and/or PEG-based resins. Functional groups suitable for immobilization of amino acids are preferably Fmoc, Boc, Nps, most preferably Fmoc.
b) the control device retrieves the sequences of peptides to be synthesized and determines the sequence specific timing protocol for each reactor;
c) the dosing device dispenses measured quantity of the first *N*-protected amino acid solution through the lifting and positioning device to the first one of the pre-activating compartments of the distribution rotor; the concentration of the *N*-protected amino acid stock solution is in the range of from 0.05 M to 0.5 M, preferably from 0.1 M to 0.4M. *N*-protecting group is selected from the group comprising tert-butyloxycarbonyl (Boc), 9H-fluoren-9-ylmethoxycarbonyl (Fmoc).
d) the distribution rotor turns to enable the second pre-activation compartment a reception of contents of the dosing device through lifting and positioning device;
e) steps c) and d) repeat at most until each pre-activation compartment contains *N*-protected amino acid solution according to the timing protocol;
f) the dosing device dispenses measured quantity of a solution of carboxyl group activating compound through the lifting and positioning device to the first one of the pre-activating compartments of the distribution rotor; the carboxyl activating compound being selected from carbodiimides and triazolols, preferably the carboxyl activating compound is diisopropylcarbodiimide. The concentration of the carboxyl activating compound stock solution is in the range of from 0.05 M to 1 M, preferably from 0.1 M to 0.5M.
g) the distribution rotor turns to enable the second pre-activation compartment a reception of contents of the dosing device through lifting and positioning device;
h) steps f) and g) repeat at most until each pre-activation compartment receives solution of carboxyl group activating compound according to the timing protocol;
i) distribution rotor is lowered to position in which it makes contact with reactors in the centrifuge;
j) the distribution rotor rotates in a synchronized rotation with the centrifuge, causing the contents of the pre-activation compartments to transfer by a centrifugal force via grooves into the reactors; the speed of the rotation being in the range of from 50 to 200 rpm.
k) distribution rotor detaches from reactors by lifting mechanism to its upper position, and reactors contents are being stirred; the stirring is performed either by the interaction of the magnets inside the reactors with magnets attached to the centrifugation drum, which causes the resin inside the reactors stir thoroughly during a slow rotation (preferably from ca 10 rpm to ca 50 rpm) of the centrifuge, or by a shaking motion of the centrifuge (fast change of the forward and backward rotation of the centrifuge);
l) condensation reaction of activated *N*-protected amino acids with functional groups suitable for immobilization of amino acids on the resin beads proceeds for predetermined time, or, alternatively, if a color changing indicator was added in one of the previous steps, until the color change indicates complete reaction; the color changing indicator may be added at the activation step h) or after the last washing step q);
m) the centrifuge with reactors rotates so that the liquid from the reactors is transferred by a centrifugal force via the S-trap outflows out of the reactors; the speed of the rotation being preferably in the range of from 500 to 1000 rpm; condensation steps c) to m) may be repeated several times, using the same or different concentrations of the particular amino acid added;
n) the dosing device dispenses measured quantity of a solvent through the lifting and positioning device to each one of the reactors of the centrifuge, and the centrifuge with reactors rotates so that the liquid from the reactors is transferred by a centrifugal force via the S-trap outflows out of the reactors; the solvent is preferably selected from the group comprising DMF, NMP, DCM. This washing step can be repeated several times (preferably 2 to 6 times) in order to wash out all the traces of non-reacted reagents from the previous step;
o) the dosing device dispenses measured quantity of a deprotection agent solution through the lifting and positioning device to each one of the reactors of the centrifuge, where a deprotection of *N*-protected end of the amino acids takes place; the time for deprotection of *N*-protected end of the amino acids depends on the protecting group and deprotecting agent used and is obvious to the skilled in the art; the deprotection agent is suitable for deprotection of the above-mentioned *N*-protecting group of amino acids, and the skilled in the art would be able to determine which deprotection agent at which concentration to use. (For example 20% piperidine in DMF requires 20 minutes to completely deprotect the Fmoc group, 20% piperidine in DMF with 2% DBU requires only 2 minutes and 100% TFA needs only one minute to completely remove Boc group.)
p) the centrifuge with reactors rotates so that the liquid from the reactors is transferred by a centrifugal force via the S-trap outflows out of the reactors; the speed of the rotation being preferably in the range of from 500 to 1000 rpm.
q) repeating step m) in order to wash the resin beads from non-reacted reagents;
r) repeating steps c) to p) for second and each following *N*-protected amino acids according to the timing protocol, until the desired peptide sequence is completed;
s) cleaving the final peptide from resin beads by a cleaving agent; the cleaving agent is suitable for cleavage of the synthesized peptide from the resin, and the skilled in the art would be able to determine which cleaving agent to use.

In one preferred embodiment, wherein the volume of the reactors is greater than ca 10 ml, the reactors content in step k) is being stirred by a slow rotation under assembly of magnets when small magnets are placed in each reactor. This ensures a rigorous stirring of larger volumes of reactor contents.

In another preferred embodiment, wherein the volume of the reactors is smaller than ca 10 ml, the reactors content in step k) is being stirred by repetitive back and forth motion of the rotor. Such movement is sufficient to stir smaller volumes of reactor contents and no magnet/magnetic stirrer needs to be placed into the reactors.

### Brief Description of Figures

Fig. 1 shows a cross-sectional view of the device for parallel oligomer synthesis in the embodiment in which the device comprises all sub-systems.
Fig. 2 shows a perspective view of the device for parallel oligomer synthesis in the embodiment in which the device comprises all sub-systems.
Fig. 3 shows a cross-sectional detail view of the reactor in which the oligomer synthesis occurs together with the S-trap outflow system.
Fig. 4 shows a cross-sectional view of the centrifugation subsystem - reactors fixed in the reactor holders and fixation slots.
Fig. 5 shows a perspective view of the centrifugation subsystem - reactors fixed in the reactor holders and fixation slots.
Fig. 6a and 6b show a top view and a perspective view of the distribution rotor.
Fig. 7 shows a cross-sectional view of the connection between the distribution rotor and the positioning mechanism and the lifting mechanism, which enable the movement of the distribution rotor.
Fig. 8 shows a perspective view connection between the distribution rotor and the positioning mechanism and the lifting mechanism, which enable the movement of the distribution rotor.
Fig. 9 shows a cross-sectional view of the centrifugation drum and the driving motor of the centrifuge.
Fig. 10 shows a perspective view of the centrifugation drum and the driving motor of the centrifuge.
Fig. 11 shows a cross-sectional view of the selector device.
Fig. 12 shows a perspective view of the selector device.
Fig. 13 shows a detailed view of the graphite layers within the selector device.
Fig. 14 shows a schematic view of the device for parallel oligomer synthesis in the embodiment in which the device comprises all sub-systems.
Fig. 15 shows a schematic view of the device for parallel oligomer synthesis in the embodiment in which the device comprises a solenoid valve in addition to all other sub-systems.
Fig. 16 shows an HPLC trace of a 21mer peptide
Fig. 17 shows a mass spectrum of a 21mer peptide
Fig. 18 shows an HPLC trace of a model YGGFL peptide

### Examples

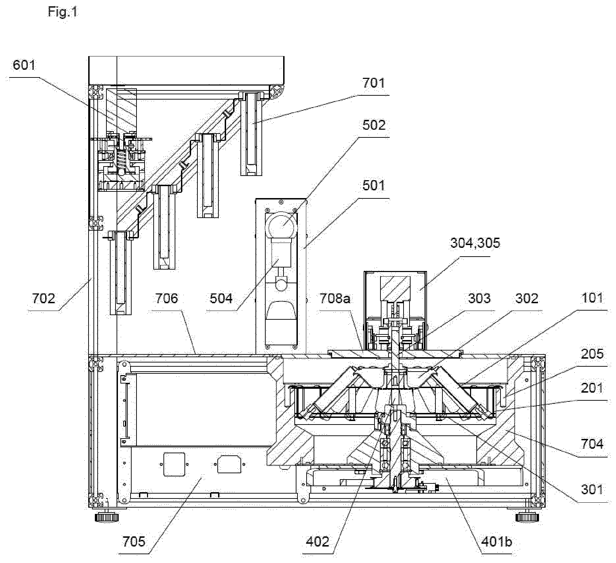

Example 1: In the following example an embodiment device for parallel oligomer synthesis will be described in the embodiment in which the device comprises all sub-systems in accordance with Fig. 1.

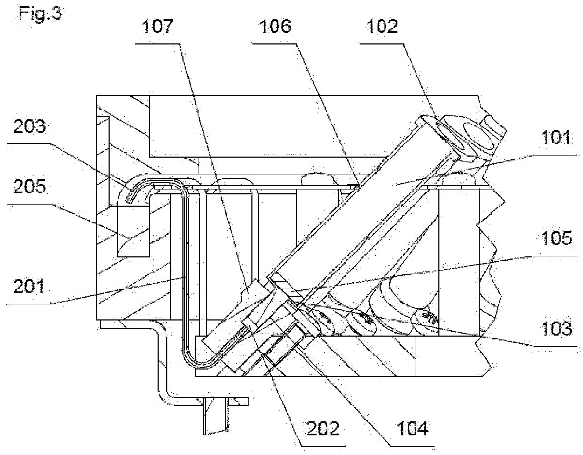

The synthesis itself takes place in reactors (Fig. 3), which are constituted by plastic syringes 101. Each of the reactors has an upper end 102 and a lower end 103. The upper end is an opened end and the lower end comprises a nozzle 104. Inside the reactor 101, a filter 105 is provided at the lower end 103 close to the nozzle 104 to provide a semipermeable membrane, which allows the resin beads, which are placed in the reactors 101 during the synthesis, to stay inside the reactor, while it allows the reagents to be washed out of the reactor 101. The filter 105 used in the example is a sintered plastic (polypropylene, Teflon), but may be also sintered glass, or metal.

The nozzles 104 of the reactors 101 are connected to S-trap outflows 201. Each of the S-trap outflows 201 has a tubular shape and comprises a first end 202 and a second end 203. The first end 202 of the S-trap outflow 201 is connected to the nozzle 104 of the lower end 103 of the reactor 101. The S-trap outflow 201 works on a siphoning principle, i.e. the second end 203 of the S-trap outflow 201 is positioned higher than the level of content in the reactor 101. The second end 203 of the S-trap outflow 201 also comprises a nozzle. The nozzle of the S-trap outflow 201 is positioned so that a liquid removed from the reactor 101 through the second end nozzle of the S-trap outflow 201 flows into a circular outflow channel 205.

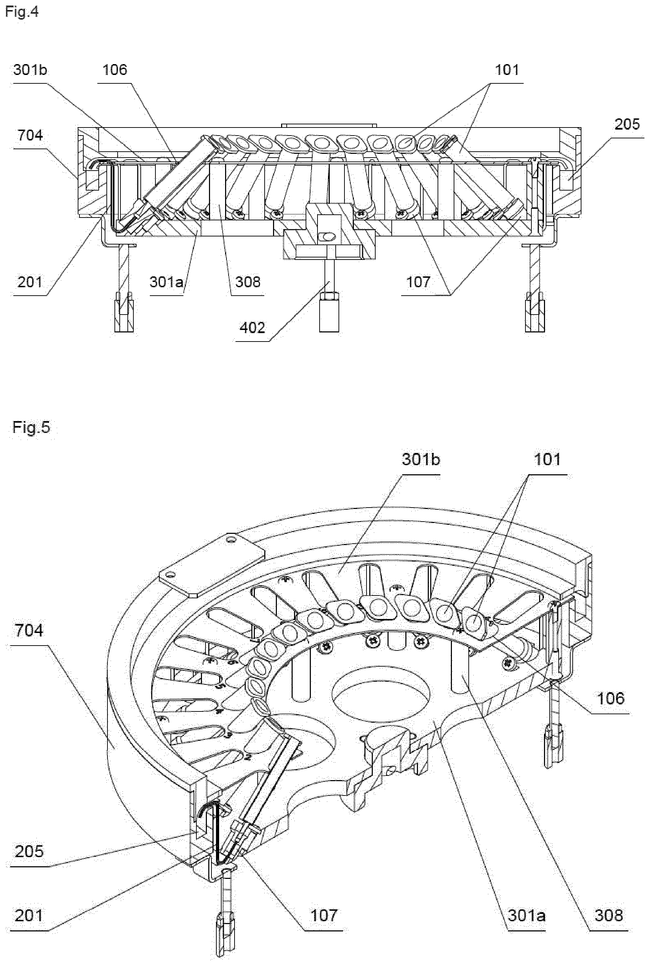

As can be seen in Figs. 4 and 5, the reactors 101 and the S-trap outflows 201 are positioned in a centrifugation subsystem comprising a centrifuge 301. The centrifuge in this example is a circular disk seated on a center axle 402. The centrifuge disk is in this example made of steel. The centrifuge is driven by a driving motor 401 connected to the axle 402 of the centrifuge 301 by a transmission 401b.

The centrifuge 301 shown in this example comprises two circular disks - a lower disk 301a and an upper disk 301b, both made of steel and seated on a common axle 402 in the center of mass. The two disks 301a, 301b are positioned vertically at a distance, but physically connected by screws 308. The upper disk 301b comprises reactor holders - openings 106, which are configured to retain the reactors 101 at an angle. The reactors 101 are positioned at an angle of 45 degrees in this example. The lower disk 301a comprises fixation slots 107, in which the nozzles 104 of the lower ends 103 of the reactors 101 are fixed. The reactors 101 are inserted from above, through the reactor holders 106 into the fixation slots 107.

The upper disk 301b is further provided with openings (not shown) for the second ends 203 of the S-trap outflows 201 to be retained in such a way that the nozzles of the second ends 203 of the S-trap outflows 201 are positioned in the circular outflow channel 205 and such that the content of the liquid in the reactor 101 reaches a level which is lower than the second end 203 of the S-trap outflow 201. In this way, the S-trap outflows 201 work on the siphoning principle, such that depending on the speed of rotation, the liquid may stay inside the reactor 101 or may be removed from it partially or completely. The liquid is removed from the reactor 101 through the nozzle of the S-trap outflow 201 and flows into the circular outflow channel 205, which is constituted by a groove positioned on the perimeter of the centrifuge 301 and aligned with the nozzles of S-trap outflows 201, such that the nozzles of S-trap outflows 201 are positioned in the circular outflow channel 205. The circular outflow channel 205 comprises outflow openings positioned on its bottom. The outflow openings of the outflow channel 205 are connected to outflow pipes, which provide an outflow system of the device 1 and drain away the liquid removed from the reactors 101 during the synthesis.

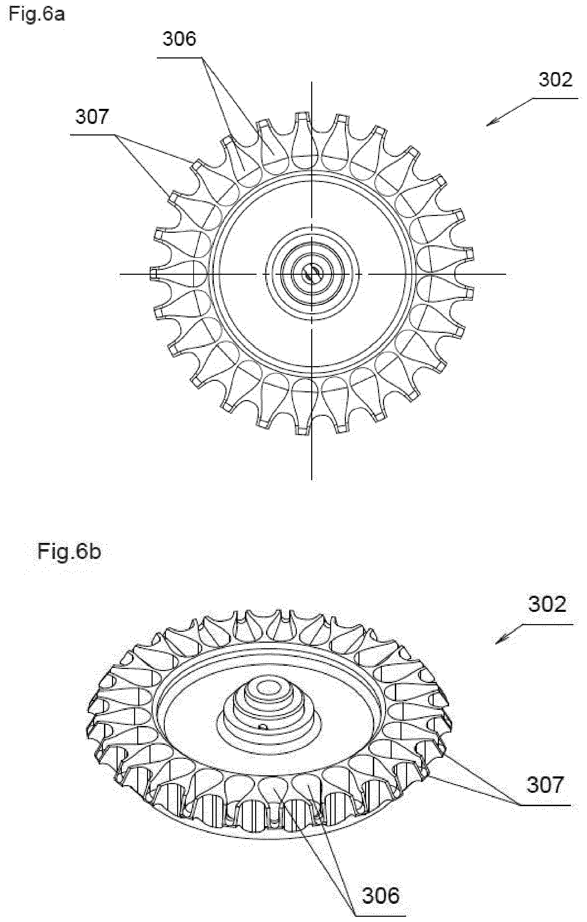

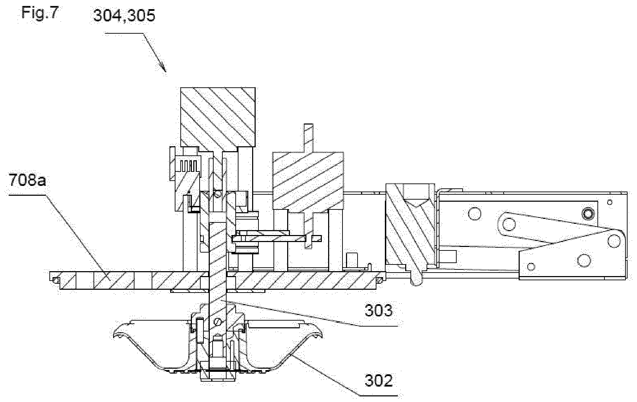

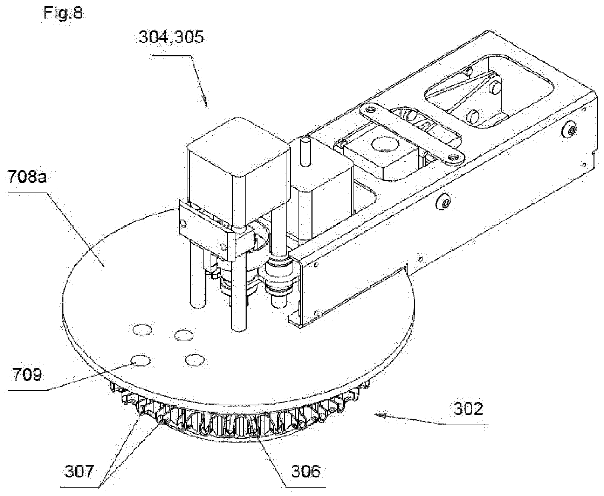

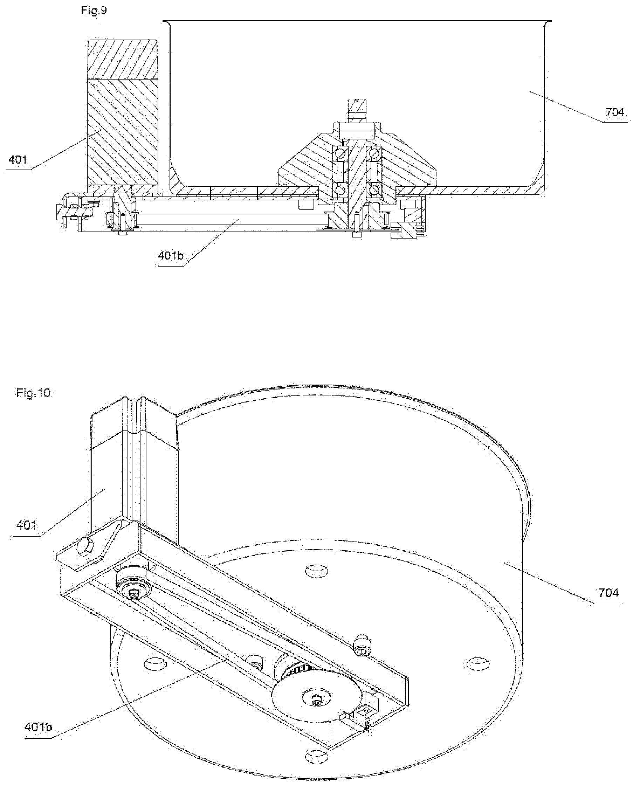

Above the centrifuge 301, a circular disk of the distribution rotor 302 made of plastic is positioned (Figs. 6a, 6b). The distribution rotor 302 and the centrifuge 301 have the same common axis in the center of mass, however, the distribution rotor 302 is seated on its own axle 303 and it is connected to a positioning device 304, i.e. a driving motor to allow positioning of the distribution rotor 302 independently from the centrifuge 301.
The distribution rotor 302 is further movable in vertical direction between a lower position and an upper position. The vertical movement is provided by a lifting device 305, which may be another motor or gas operated piston. In this example, the stepper motor is provided as the lifting device. The positioning and the lifting device can be seen in Figs. 7 and 8.
The distribution rotor 302 is driven by its own driving motor (positioning device 304) only when it is positioned in the upper position, when it is detached from the centrifuge 301. When the distribution rotor 302 is positioned in the lower position, the motor driving the distribution rotor (positioning device 304) is disengaged from the distribution rotor 302 and all actuation of the distribution rotor 302 is performed by the motor 401 driving the centrifuge 301 (Figs. 9, 10) so that the synchronized rotation of the centrifuge 301 and the distribution rotor 302 is ensured.

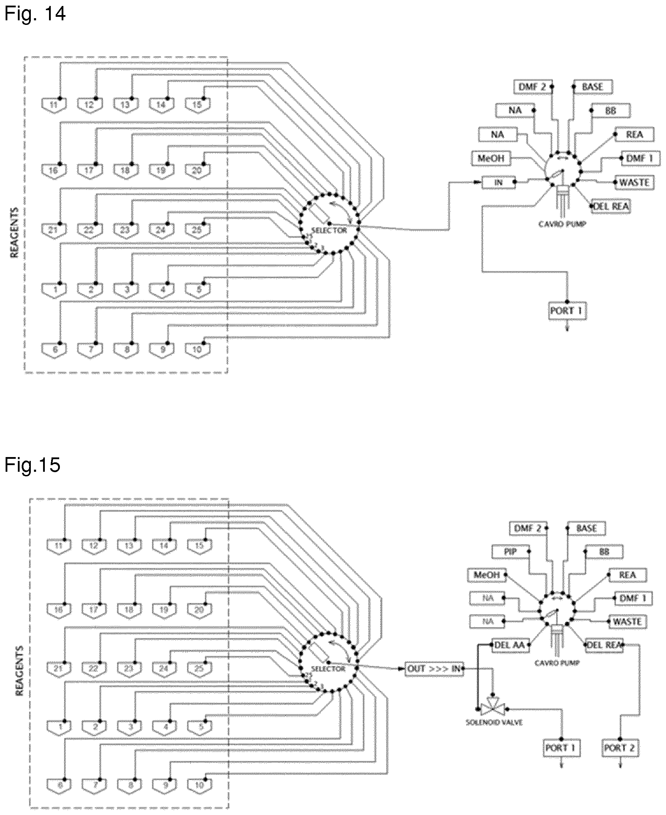

The distribution rotor 302 comprises pre-activation compartments 306, which are positioned at the outer circumference of the distribution rotor 302, and which are provided with grooves 307 directed radially outwards, towards the outer edge of the distribution rotor 302. These grooves 307 enable the connection between the pre-activation compartments 306 of the distribution rotor 302 and centrifuge 301 when the distribution rotor 302 is in the lower position such that the contents of the pre-activation compartments 306 is transferred to the reactors 101 upon synchronized rotation of the distribution rotor 302 and the centrifuge 301 at medium rotation speed.
In this example, the pre-activation compartments 306 are filled in via a dosing device 501 (shown schematically in Figs. 14, 15). The dosing device 501 comprises a distribution valve 502 and a dosing pump 504. The dosing pump 504, which is in this example a programmable syringe pump and is configured to measure the exact quantity of the liquid reagent to be dosed into the individual preactivation compartments, is connected to the distribution valve 502. The distribution valve 502 comprises a plurality of ports, 12 ports in this example. The distribution valve 502 comprises at least one outtake port which is via tubing (not shown in figures) connected to the centrifugation subsystem (distribution rotor 302) and through which the individual pre-activation compartments 306 are filled in through the inlet port 709. The other ports of the distribution valve 502 are intake ports and provide for a connection with stock solutions of reactants and/or solvents and/or activating reagents and/or coupling reagents stored in storage containers placed in locations 710.
The dosing pump 504 is connected to the desired reactant via one of the ports of the distribution valve 502 to make the intake connection (while the other ports are closed) and a desired quantity may be sucked by the dosing pump 504 inside the dosing pump 504. Subsequently, the intake connection may be closed and an outtake connection between the dosing pump 504 and the distribution rotor 302 may be filled through the inlet port 709. In this way, the desired quantity of the reactant is transferred via tubing, and one of the pre-activation compartments 306 is filled in. This process is repeated with either the same reactant or other reactants until the desired number of pre-activation compartments 306 is filled in.

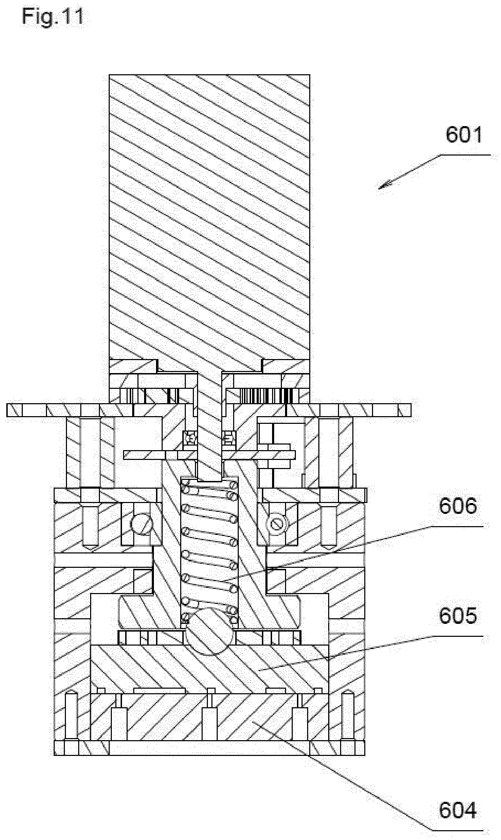

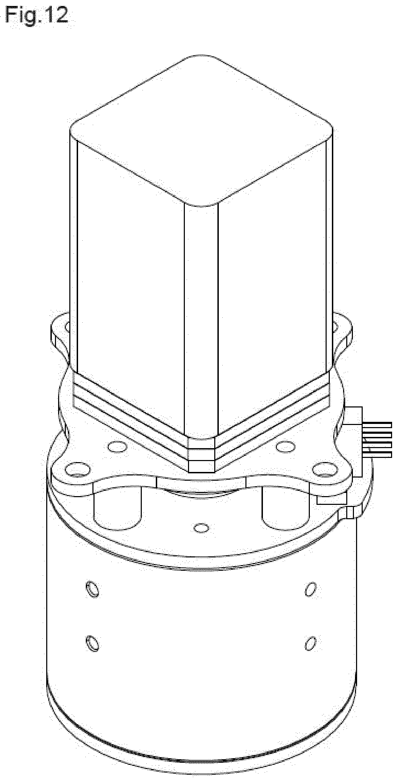

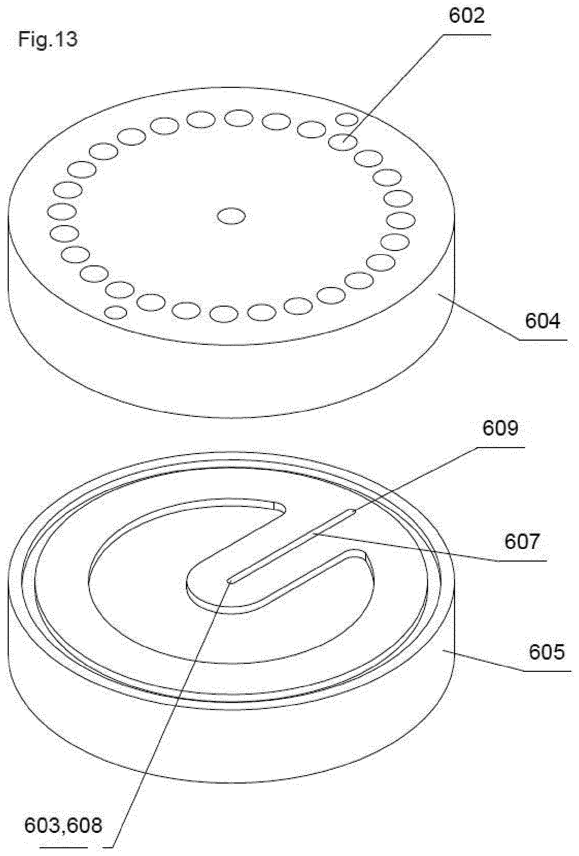

In this example, one of the intake ports of the distribution valve 502 is connected to a selection device 601 (Figs. 11, 12), which is a valve configured to select the desired amino-acid. The selected amino acid is then transferred through the selector device 601, the distribution valve 502 and the dosing pump 504 into the individual pre-activation compartments 306 of the distribution rotor 302. The selection device 601 comprises a plurality of intake ports 602; 28 intake ports in this example. The intake ports are connected via tubing with the storage containers 701 containing various amino-acids. The selection device 601 further comprises an outtake port 603, which is connected via tubing with one of the intake ports of the distribution valve 502 of the dosing device 501. In order to retrieve the desired amino-acid from one of the storage containers 701, the connection between the dosing pump 504, one of the intake ports of the distribution valve 502, the outtake port 603 of the selector device 601 and one of the intake ports 602 of the selector device 601 is opened and the desired quantity of the desired amino-acid is sucked from the storage container 701 into the dosing pump 504. The desired quantity of the desired amino-acid is then transferred from the dosing pump 504 through the outtake port of the distribution valve 502 into one of the pre-activation compartments 306.
In this example, the selection device 601 comprises two coplanar, circular layers (604,605) of graphite pressed together by spring 606. The upper layer 604 comprises the plurality of intake ports 602, located close to the outer circumference of the upper layer. The lower graphite layer 605 comprises the outtake port 603 and an elongated groove 607. The outtake port 603 is located in the middle of the circular graphite layer 605. The elongated groove 607 has a first end 608 and a second end 609. The position of the first end 608 of the groove 607 is identical with the position of the outtake port 603. The groove 607 is elongated radially outwards from the position of the first end 608 of the groove 607 such that the position of the second end 609 of the elongated groove 607 is identical with position of one of the intake ports 602 in the upper graphite layer 604. As the intake ports 602 are positioned in a circular configuration along the outer circumference of the upper graphite layer 604, the elongated groove 607 allows to establish a connection between the outtake port 603 and any one of the intake ports 602 by rotating of one of the two coplanar graphite layers (604, 605) relatively to the other one. In order to ensure the non-crosscontamination of the desired reactant by the reactant from any other unselected port, this coplanar arrangement selector port is used in underpressure (suction) regimen, when the sealing of the two coplanar surfaces can be guaranteed. The detailed view of the coplanar structure of the selection device 601 is shown in Fig. 13.
The solutions of amino-acids are in this example stored in storage containers 701. The number of storage containers corresponds to the number of intake ports 602 of the selector device 601, i.e. 28 in this example. The storage containers 701 have unified cylindrical shape and are placed in a holder 702. The holder 702 has means for holding the storage containers 701.

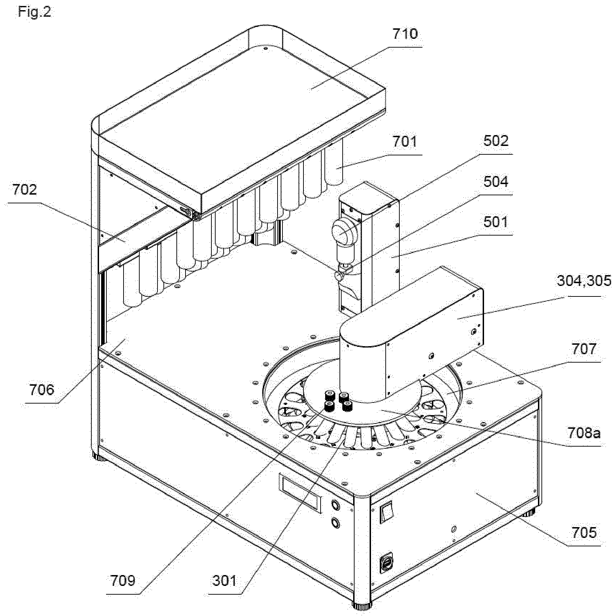

As shown in Figs. 1 and 2, the centrifuge 301 and the distribution rotor 302 are placed in a centrifugation drum 704 of a cylindrical shape with diameter slightly larger than the diameter of the centrifuge 301, such that the centrifuge 301 fits in the centrifugation drum 704. The centrifugation drum 704 is placed in a box 705, which has an upper part 706 with a circular opening 707 in this upper part 706, into which the centrifugation drum 704 is placed. In this example, the upper part 706 of the box 705 serves as a storage place for the dosing device 501. The holder 702 with the selector device 601 and the storage containers 701 is also attached to the upper part 706 of the box 705. The box 705 further retains the outflow pipes, which provide an outflow system of the device and drain away the liquid removed from the reactors 101 during the synthesis. These outflow pipes are combined into one outflow tube, which is positioned within the box 705.

The centrifugation subsystem is covered in the centrifugation drum 704 by a cover 708, which is rotatably attached to the upper part 706 of the box 705 and moves rotatably from an open position to a closed position and vice versa. The cover 708 is in this example comprises two concentric disks - the inner disk 708a is made of polypropylene and the outer disk 708b is made of glass to allow the operator to watch the operation of the device directly. The inner disk 708a of the cover is placed between the distribution rotor 302 and the positioning device 304 (and the lifting device 305), as both of them are placed above the distribution rotor 302 and outside of the centrifugation drum 704. There is at least one opening - inlet port 709 in the inner disk 708a of the cover 708, through which the distribution rotor 302 of the centrifugation subsystem may be connected to the dosing device 501.

The system is controlled by a timing protocol of the program constructed for operation of the device 1, such that all sub-devices are connected to that control subsystem and operated by the timing protocol of the control subsystem.

Example 2: In another embodiment, the device 1 for parallel oligomer synthesis comprises a distribution device (not shown) in addition to the components as described in Example 1. The distribution device connects the dosing pump 504 via the distribution valve 502 to either the selector device 601 or to the pre-activation compartments 306 of the distribution rotor 302. The distribution device is a solenoid valve with three ports switching between the connection of the dosing pump 504 with the outtake port of selector device 601 and the connection between the dosing pump 504 and the pre-activation compartments 306 of the distribution rotor 302 (through the distribution valve 502).

In this example, when the distribution device is enabled in the system, the distribution valve 502 comprises 12 ports for connection with the distribution device.

It is also possible to place a tubing with a predefined volume (which is larger than the volume being delivered to the reactor) between the dosing pump 504 and the inlet port 709 of the pre-activation compartments 306 to avoid the reagents entering the dosing pump 504 inner volume. In this arrangement, the distribution device is placed between selector device 601 and inlet port 709 of the centrifuge assembly. The distribution device is first opened in the direction to the dosing pump 504, the liquid is sucked from the distribution valve 502 into the tubing, the distribution device is opened in direction of delivery to one of the reactors 101 and the liquid is expelled into the reactor, without entering the syringe of the dosing pump 504 and contaminating the liquid in there. This schematic arrangement of this example can be seen in Fig. 15.

Example 3: In this example, the device 1 may further (in addition to the components described in the examples above) comprise a temperature control device, provided inside the centrifugation drum 704 (not shown in the figures). The temperature control device in this example comprises an infrared radiator and a microwave radiator, and a sensor with a feedback control. Such a temperature control device may provide temperatures up to 90 degrees Celsius and is controlled automatically by the control subsystem.

Example 4: In another example (not shown in the figures), the device 1 may further (in addition to the components described in the examples above) comprise a first plurality of magnets which are attached statically to the inner side of the centrifugation drum 704 above the reactors 101, such that they are distributed along the circumference of the centrifugation drum 704. Further, a second plurality of magnets is positioned inside each of the reactors 101. The size of the magnets placed inside the reactors 101 is smaller than the size of the magnets attached to the centrifugation drum 704. Because of the interaction of the magnets inside the reactors 101 with magnets attached to the centrifugation drum 704, the resin inside the reactors 101 is enabled to be mixed more thoroughly during the slow rotation of the centrifuge 301 in the synthetic process. The addition of magnets is especially advantageous for larger quantities of contents inside the reactor, when rotation and/or shaking of the centrifuge is not sufficient to mix the content to a desired extent.

Example 5: *Synthesis of ValA10-insulin A-chain S-sulfonate (Mw 2687.8116 and 2689.9100)* Sequence (21 residues): Gly-Ile-Val-Glu-Gln-Cys(SSO3H)-Cys-Thr-Ser-Ile-Cys(SSO3H)-Ser-Leu-Tyr-Gln-Leu-Glu-Asn-Tyr-Cys(SSO3H)-Asn was synthesised using the device according to the present invention and the method according to the present invention. Preloaded Fmoc-Asn(Trt)-Wang-LL-resin (0.29 mmol/g), 100 µmol scale, was used. The resin was purchased from IRIS Biotech GmbH. Resin was placed to two PP syringes of the 10 ml volume with frits (2x 50 µmol of the Fmoc groups).
The control device retrieved the sequence of Gly-Ile-Val-Glu-Gln-Cys(SSO3H)-Cys-Thr-Ser-Ile-Cys(SSO3H)-Ser-Leu-Tyr-Gln-Leu-Glu-Asn-Tyr-Cys(SSO3H)-Asn and determined the sequence specific timing protocol. Two couplings per position were used (each condensation was performed twice for each step). The first coupling (45 min) was done with 1 ml of 0.5 M amino acid and 0.5 ml of 0.5M DIC in DMF. The second coupling (45 min) was done with 0.5 ml of 0.5 M amino acid and 0.5 ml of 0.5M DIC in DMF.
The dosing device dispensed 1 ml of first *N*-protected 0.5 M amino acid solution through the lifting and positioning device to the first one of the pre-activating compartments of the distribution rotor, the distribution rotor turned to enable the second pre-activation compartment a reception of contents of the dosing device through lifting and positioning device. The dosing device dispensed 0.5 ml of 0.5 M solution of diisopropylcarbodiimide (DIC) through the lifting and positioning device to the first one of the pre-activating compartments of the distribution rotor, the distribution rotor turned to enable the second pre-activation compartment a reception of contents of the dosing device through lifting and positioning device and the steps repeated to ensure the reception of 0.5 ml of 0.5 M solution of diisopropylcarbodiimide (DIC) to the second one of the pre-activating compartments of the distribution rotor according to the timing protocol.
The distribution rotor was lowered to position in which it makes contact with reactors in the centrifuge, and rotated with a speed of 100 rpm in a synchronized rotation with the centrifuge, causing the contents of the pre-activation compartments to transfer by a centrifugal force via grooves into the reactors. Then the distribution rotor detached from reactors by lifting mechanism and reactors content were stirred by repetitive back and forth motion for 45 minutes in order to complete the condensation reaction.
Then the centrifuge with reactors rotated with the speed of 1000 rpm so that the liquid from the reactors was transferred by a centrifugal force via the S-trap outflows out of the reactors.
The dosing device dispensed 1 ml of DMF through the lifting and positioning device to each one of the reactors of the centrifuge, and the centrifuge with reactors rotated with the speed of 1000 rpm so that the liquid from the reactors was transferred by a centrifugal force via the S-trap outflows out of the reactors. This washing step was repeated 5 times.
In the next step, the dosing device dispensed 2 ml of 20% piperidine/2% DBU in DMF through the lifting and positioning device to each one of the reactors of the centrifuge, where a deprotection of *N*-protected end of the amino acids took place for 2 minutes before the centrifuge with reactors rotated so that the liquid from the reactors was transferred by a centrifugal force via the S-trap outflows out of the reactors. Then washing step occurred using 5 times 1 ml DMF as described above.

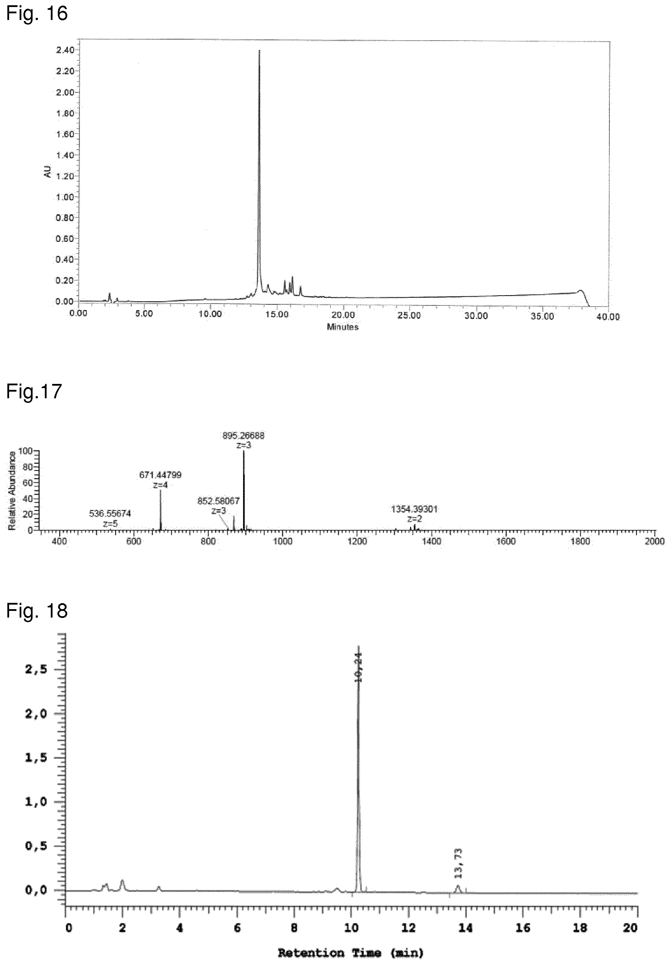

The above described procedure was repeated for the second and the following amino acids in the synthesized peptide sequence, until the desired peptide sequence was completed. The final peptide was cleaved from the resin using TFA/scavengers and precipitated in a cold diethyl ether. The precipitate, crude peptide in SH form, was immediately converted to S-sulfonate derivative using a protocol described in Kosinova et al. Biochemistry 2014, 53, 3392-3402. HPLC trace of the crude S-sulfonate product (178 mg) is shown in Figure 16.
The identity of the product main in the main peak was confirmed by MS (negative mode, see Figure 17). All main m/z signals in the spectrum belong to the product.

Example 6: *Synthesis of YGGFL mutants*
Sequences (24 x YGGFL mutants): YGGFL, GGYFL, GGYLF, GGFYL, GGFLY, GGLYF, GGLFY, GYGFL, GYGLF, GYFGL, GYFLG, GYLGF, GYLFG, GFGYL, GFGLY, GFYGL, GFYLG, GFLGY, GFLYG, GLGYF, GLGFY, GLYGF, GLYFG, YGGFL
Rotor size 25 positions
Number of couplings per position: 1
Centrifugation time 20 sec
Coupling time 30 min

Resin Fmoc-Rink amide resin s=0.5 mmol/g, 24x 20 mg
Fmoc-AA/oxyma/DMF volume 0.4 mL
Fmoc-AA/oxyma molarity 0.5
Reagent (DIC)/DMF molarity 1.0
Reagent volume 0.24 mL

Sequences (24 x YGGFL mutants): YGGFL, GGYFL, GGYLF, GGFYL, GGFLY, GGLYF, GGLFY, GYGFL, GYGLF, GYFGL, GYFLG, GYLGF, GYLFG, GFGYL, GFGLY, GFYGL, GFYLG, GFLGY, GFLYG, GLGYF, GLGFY, GLYGF, GLYFG, YGGFL were synthesised using the device according to the present invention and the method according to the present invention.
Fmoc-Rink amide resin s=0.5 mmol/g, 24x 20 mg was placed into the PP syringes with frits (24x 20 mg) and swollen in DMF. The resin was purchased from IRIS Biotech GmbH.
The control device retrieved the sequence of first of the sequences above and determined the sequence specific timing protocol. Single coupling was applied using oxyma/DIC activation.

The dosing device dispensed 0.4 ml of first *N*-protected 0.5 M amino acid solution through the lifting and positioning device to the first one of the pre-activating compartments of the distribution rotor, the distribution rotor turned to enable the second pre-activation compartment a reception of contents of the dosing device through lifting and positioning device. The dosing device dispensed 0.24 ml of 1.0 M solution of diisopropylcarbodiimide (DIC) through the lifting and positioning device to the first one of the pre-activating compartments of the distribution rotor, the distribution rotor turned to enable the second pre-activation compartment a reception of contents of the dosing device through lifting and positioning device and the steps repeated to ensure the reception of 0.24 ml of 1.0 M solution of diisopropylcarbodiimide (DIC) to the second one of the pre-activating compartments of the distribution rotor according to the timing protocol.
The distribution rotor was lowered to position in which it makes contact with reactors in the centrifuge, and rotated with a speed of 100 rpm in a synchronized rotation with the centrifuge, causing the contents of the pre-activation compartments to transfer by a centrifugal force via grooves into the reactors. Then the distribution rotor detached from reactors by lifting mechanism and reactors content were stirred by repetitive back and forth motion for 30 minutes in order to complete the condensation reaction.
Then the centrifuge with reactors rotated with the speed of 1000 rpm for 20 s so that the liquid from the reactors was transferred by a centrifugal force via the S-trap outflows out of the reactors.
The dosing device dispensed 1 ml of DMF through the lifting and positioning device to each one of the reactors of the centrifuge, and the centrifuge with reactors rotated with the speed of 1000 rpm so that the liquid from the reactors was transferred by a centrifugal force via the S-trap outflows out of the reactors. This washing step was repeated 2 times.
In the next step, the dosing device dispensed 1 ml of 20% piperidine/2% DBU in DMF through the lifting and positioning device to each one of the reactors of the centrifuge, where a deprotection of *N*-protected end of the amino acids took place for 2 minutes before the centrifuge with reactors rotated so that the liquid from the reactors was transferred by a centrifugal force via the S-trap outflows out of the reactors. Then washing step occured using 5 times 1 ml DMF as described above.
The above described procedure was repeated for the second and the following aminoacids in the synthesised peptide sequence, until the desired peptide sequence was completed. After completion of the synthesis, the resin was washed with methanol (2x) and dried. The final peptide was cleaved from the resin by manual addition of 95%TFA/5% H₂O (200 µL per reactor) for 2 hrs.
HPLC analysis was performed after dilution of the sample with 800 µL of water.
An example of HPLC trace of the crude product is shown in Figure 18. (H-YGGFL-NH₂, column Symmetry C₁₈ 3.5 µm, 4.6 x 75 mm, grad 5-65% MeCN/20 min).

## Claims

1. A device for parallel oligomer synthesis, the device comprising:
a centrifuge (301), the centrifuge comprising a plurality of reactor holders (106), the reactor holders being configured to retain reactors at an angle, and
a plurality of S-trap outflow holders,
a plurality of reactors (101), the reactors having upper ends and lower ends, the lower ends comprising nozzles, the reactors being positioned at an angle in the reactor holders,
a plurality of S-trap outflows (201), the S-trap outflows having first ends and second ends, the first ends of the S-trap outflows being connected to the nozzles of the lower ends of reactors, and the S-trap outflows being configured to work on a siphoning principle,
a distribution rotor (302), the distribution rotor being provided with pre-activation compartments (306) at its outer circumference, the pre-activation compartments being provided with grooves directed towards the outer edge of the distribution rotor, and configured to enable the transfer of contents from the pre-activation compartments of the distribution rotor to the reactors upon rotation of the distribution rotor,
a lifting device (305), the lifting device being configured to enable the distribution rotor to move in vertical direction between an upper position and a lower position,
a positioning device, the positioning device being configured to enable a rotation of the distribution rotor in the upper position independently from the centrifuge,
a driving motor, the driving motor, the centrifuge and the distribution rotor having the same common axis, the driving motor being configured to enable a synchronized rotation of the centrifuge and the distribution rotor in the lower position, an outflow channel (205), the outflow channel being positioned at the outer circumference of the centrifuge, the second ends of the S-trap outflows being positioned in said outflow channel continuous groove, the outflow channel further comprising a plurality of outflow openings, a system of outflow pipes, the outflow pipes being connected to outflow openings of the outflow channel and being positioned below the centrifuge,
a dosing device (501), the dosing device comprising a distribution valve (502), the distribution valve having a plurality of ports, and a dosing pump (504), the dosing pump being configured to measure the exact quantity of liquid reagents
the dosing pump being connected to the distribution valve,
the distribution valve being connected to stock solutions of reactants and/or solvents and/or activating reagents and/or coupling reagents via plurality of ports of the distribution valve and via tubing, and
a control device, the control device being provided with a timing protocol and connected to said driving motor, said dosing device and said lifting and positioning device.

2. A device for parallel oligomer synthesis according to claim 1, wherein the centrifuge further comprises:
a first disk made of inert material, the first disk being provided with the reactor holders on its inner circumference, and with a second set of openings for the S-trap outflows on its outer circumference,
a second disk made of inert material, the second disk being seated on the same axle as the first disk and positioned below the first disk, and provided with fixation slots for fixing the reactors and with a first set of openings for S-trap outflows on its outer circumference,
such that the plurality of reactors are situated in the reactor holders of the first disk and fixed in the fixation slots of the second disk, and
a plurality of S-trap outflows are connected to the plurality of reactors through the first set of openings in the second disk and fixed by the second set of openings in the first disk.

3. The device for parallel oligomer synthesis according to any one of the preceding claims further comprising a selector device, and
a plurality of storage containers for storing reagents, wherein
the selector device is connected to the plurality of storage containers and to the dosing device via tubing.

4. The device according to claim 3, wherein the selector device comprises:
coplanar layers of graphite pressed together by spring,
plurality of intake ports connecting the selector device with the plurality of storage containers, and
an outtake port connecting the selector device with the dosing device.

5. The device for parallel oligomer synthesis according to claims 3 or 4 further comprising a distribution device, the distribution device being connected via tubing to the selector device, dosing device and to the distribution rotor, the distribution device being preferably a solenoid valve connecting the dosing device with either the selector device or the distribution rotor.

6. The device for parallel oligomer synthesis according to any one of the preceding claims, wherein the reactors are syringes, preferably made of plastic, and wherein the syringes comprise filters, the filters preferably made of sintered glass, plastic or metal mesh, or any porous material.

7. The device for parallel oligomer synthesis according to any one of the preceding claims, wherein it further comprises a temperature control device, the temperature control device comprising an infrared radiator, microwave radiator, and temperature sensor with feedback control.

8. The device for parallel oligomer synthesis according to any one of the preceding claims further comprising
a plurality of magnets, the plurality of magnets being attached to the inner side of the centrifugation drum, and
a plurality of small magnets to be placed inside the reactors, in order to stir the contents of the reactors upon rotor movements.

9. The device for parallel oligomer synthesis according to any one of the preceding claims, wherein the dosing device is a programmable syringe pump.

10. The device for parallel oligomer synthesis according to any one of the preceding claims further comprising:
a centrifugation drum for placing the centrifuge, and
a box, the box having a circular opening for placing the centrifugation drum and the system of outflow pipes.

11. The device for parallel oligomer synthesis according to claim 10 further comprising:
a sealed cover positioned over the centrifugation drum, the sealed cover having a first part and
a second part, the first part made of glass or plexiglass, and the second part made of the same inert material as the centrifuge.

12. A method of parallel solid-based peptide synthesis, using the device for parallel oligomer synthesis according to any one of the claims 1 to 11, and following the timing protocol of the device, **characterized in that** it comprises the following steps:
a) resin beads provided with functional groups suitable for immobilization of amino acids are placed in the reactor; eventually equipped with a magnet placed inside the reactor;
b) the control device retrieves the sequence of the peptide to be synthesized and determines the sequence specific timing protocol;
c) the dosing device dispenses measured quantity of first *N*-protected amino acid through the lifting and positioning device to the first one of the pre-activating compartments of the distribution rotor;
d) the distribution rotor turns to enable the second pre-activation compartment a reception of contents of the dosing device through lifting and positioning device;
e) steps c) and d) repeat at most until each pre-activation compartment contains *N*-protected amino acid solution according to the timing protocol;
f) the dosing device dispenses measured quantity of a solution of carboxyl group activating compound through the lifting and positioning device to the first one of the pre-activating compartments of the distribution rotor;
g) the distribution rotor turns to enable the second pre-activation compartment a reception of contents of the dosing device through lifting and positioning device;
h) steps f) and g) repeat at most until each pre-activation compartment receives solution of carboxyl group activating compound according to the timing protocol; furthermore, color changing indicator may be added in this step for indication of the completion of the condensation step 1);
i) distribution rotor is lowered to position in which it makes contact with reactors in the centrifuge;
j) the distribution rotor rotates in a synchronized rotation with the centrifuge, causing the contents of the pre-activation compartments to transfer by a centrifugal force via grooves into the reactors;
k) distribution rotor detaches from reactors by lifting mechanism and reactors content is being stirred;
l) condensation reaction of activated *N*-protected amino acids with functional groups suitable for immobilization of amino acids on the resin beads proceeds for predetermined time, or, alternatively, if color changing indicator was added, until the color change indicates complete reaction;
m) the centrifuge with reactors rotates so that the liquid from the reactors is transferred by a centrifugal force via the S-trap outflows out of the reactors;
n) the dosing device dispenses measured quantity of a solvent through the lifting and positioning device to each one of the reactors of the centrifuge, and the centrifuge with reactors rotates so that the liquid from the reactors is transferred by a centrifugal force via the S-trap outflows out of the reactors;
o) the dosing device dispenses measured quantity of a deprotection agent solution through the lifting and positioning device to each one of the reactors of the centrifuge, where a deprotection of *N*-protected end of the amino acids takes place;
p) the centrifuge with reactors rotates so that the liquid from the reactors is transferred by a centrifugal force via the S-trap outflows out of the reactors;
q) repeating step m) in order to wash the resin beads from non-reacted reagents; furthermore, color changing indicator may be added in this step for indication of the completion of the condensation step 1);
r) repeating steps c) to p) for second and following *N*-protected amino acids according to the timing protocol, until the desired peptide sequence is completed;
s) cleaving the final peptide from resin beads by a cleaving agent.

13. The method according to claim12, wherein, in the step k) reactors content is being stirred by repetitive back and forth motion of the rotor or by a slow rotation under assembly of magnets when small magnets are placed in each reactor.

14. The method according to claiml2 or 13, wherein the step n) is repeated, preferably it is repeated at least twice, more preferably step n) is repeated 5 times.

15. Use of the device for parallel oligomer synthesis according to any one of the claims 1 to 11 in peptide synthesis.

## Patentansprüche

1. Ein Apparat zur parallelen Oligomersynthese, wobei der Apparat besteht aus:
einer Zentrifuge (301), wobei die Zentrifuge beinhaltet
eine Vielzahl von Reaktorhaltern (106),
wobei die Reaktorhalter so konfiguriert sind, das sie die Reaktoren in einem Winkel halten, und eine Vielzahl von S-Trap-Abflusshaltern,
eine Vielzahl von Reaktoren (101), wobei die Reaktoren obere Enden und untere Enden haben, wobei die unteren Enden Düsen umfassen, wobei die Reaktoren in einem Winkel in den Reaktorhaltern angeordnet sind,
eine Vielzahl von S-Trap-Abflüsse (201), wobei die S-Trap-Abflüsse erste und zweite Enden aufweisen, wobei die ersten Enden der S-Trap-Abflüsse mit den Düsen der unteren Enden der Reaktoren verbunden sind und die S-Trap-Abflüsse so konfiguriert sind, um nach einem Heberleitungssystem zu arbeiten,
einen Verteilungsrotor (302), wobei der Verteilungsrotor an seinem Außenumfang mit Voraktivierungskammern (306) versehen ist;
die Voraktivierungskammern sind mit Rillen versehen, die auf die Außenkante des Verteilungsrotors gerichtet sind und so konfiguriert sind, dass sie bei Drehung des Verteilungsrotors die Übertragung vom Inhalt der Voraktivierungskammern des Verteilungsrotors zu den Reaktoren ermöglichen,
eine Hebevorrichtung (305), wobei die Hebevorrichtung konfiguriert ist, um es dem Verteilungsrotor zu ermöglichen, sich in vertikaler Richtung zwischen einer oberen Position und einer unteren Position zu bewegen;
eine Positioniervorrichtung, wobei die Positionierungsvorrichtung konfiguriert ist, um eine Drehung des Verteilungsrotors in der oberen Position unabhängig von der Zentrifuge zu ermöglichen,
einen Antriebsmotor, den Antriebsmotor, die Zentrifuge und der Verteilungsrotor haben dieselbe gemeinsamen Achse, wobei der Antriebsmotor so konfiguriert ist, das eine synchronisierte Drehung der Zentrifuge und des Verteilungsmotors in der unteren Position möglichen ist,
ein Abflusskanal (205), wobei der Abflusskanal am Außenumfang der Zentrifuge positioniert ist, die zweiten Enden der S-Trap-Abflüsse sind so positioniert, dass sind die durchgehende Nut des Abflusskanals,
eine Dosiervorrichtung (501), wobei die Dosiervorrichtung umfasst:
ein Verteilerventil (502), wobei das Verteilerventil mehrere Öffnungen aufweist, und
eine Dosierpumpe (504), wobei die Dosierpumpe konfiguriert ist, um die genaue Menge an flüssigen Reagenzien zu messen, wobei die Dosierpumpe mit dem Verteilungsventil verbunden ist;
wobei das Verteilungsventil über mehrere Anschlüsse und über Schläuche mit Stammlösungen von Reaktanten und/oder Lösungsmitteln und/oder Aktivierungsreagenzien und/oder Kopplungsreagenzien verbunden ist; und
eine Steuervorrichtung, wobei die Steuervorrichtung mit einem Zeitsteuerungsprotokoll versehen und mit dem Antriebsmotor, der Dosiervorrichtung und der Hebe- und Positionierungsvorrichtung verbunden ist.

2. Der Apparat zur parallelen Oligomersynthese nach Anspruch 1, wobei die Zentrifuge ferner umfasst:
eine erste Scheibe aus inertem Material, wobei die erste Scheibe an ihrem Innenumfang mit den Reaktorhaltern versehen ist und an ihrem Außenumfang ein zweites Set Öffnungen für die S-Trap-Abflüsse vorhanden ist;
eine zweite Scheibe aus inertem Material, wobei die zweite Scheibe auf derselben Achse wie die erste Scheibe sitzt und unter der ersten Scheibe positioniert ist und mit Befestigungsschlitzen zum Befestigen der Reaktoren und mit einem ersten Set Öffnungen für die S-Trap-Abflüsse an seinem äußerer Umfang versehen ist,
so dass die Vielzahl der Reaktoren in den Reaktorhaltern der ersten Scheibe angeordnet und in den Fixierungsschlitzen der zweiten Scheibe fixiert sind, und
die mehrere S-Trap-Abflüsse sind mit dem ersten Set von Öffnungen in der zweiten Scheibe mit der Vielzahl von Reaktoren verbunden und durch das zweite Set von Öffnungen in der ersten Scheibe fixiert.

3. Der Apparat zur parallelen Oligomersynthese nach einem der vorhergehenden Ansprüche, wobei der Apparat umfasst ferner eine Auswahlvorrichtung, und
eine Vielzahl von Lagerbehältern zum Lagern von Reagenzien, wobei
die Auswahlvorrichtung über Schläuche mit mehreren Vorratsbehältern und mit der Dosiervorrichtung verbunden ist.

4. Der Apparat nach Anspruch 3, wobei die Auswahlvorrichtung umfasst:
koplanare Graphitschichten, die mit Federn zusammengedrückt werden,
mehrere Einlassöffnungen, die die Auswahlvorrichtung mit mehreren Vorratsbehältern verbinden, und
einen Auslassanschluss, der die Auswahlvorrichtung mit der Dosiervorrichtung verbindet.

5. Der Apparat zur parallelen Oligomersynthese nach den Ansprüchen 3 oder 4, ferner umfassend eine Verteilungsvorrichtung, wobei die Verteilungsvorrichtung über einen Schlauch mit der Auswahlvorrichtung, der Dosiervorrichtung und dem Verteilungsrotor verbunden ist, wobei die Verteilungsvorrichtung vorzugsweise ein Magnetventil ist, das die Dosiervorrichtung verbindet entweder mit der Wählvorrichtung oder dem Verteilerrotor.

6. Der Apparat zur parallelen Oligomersynthese nach einem der vorhergehenden Ansprüche, wobei die Reaktoren Spritzen sind, vorzugsweise aus Kunststoff, und wobei die Spritzen Filter umfassen, die Filter vorzugsweise aus gesintertem Glas, Kunststoff oder Metallgitter oder einem beliebigen porösen Material bestehen .

7. Der Apparat zur parallelen Oligomersynthese nach einem der vorhergehenden Ansprüche, wobei er ferner eine Temperatursteuervorrichtung umfasst, wobei die Temperatursteuervorrichtung einen Infrarotstrahler, einen Mikrowellenstrahler und einen Temperatursensor mit Rückkopplungssteuerung umfasst.

8. Der Apparat zur parallelen Oligomersynthese nach einem der vorhergehenden Ansprüche, ferner umfassend
mehrere Magnete, wobei diese Magnete an der Innenseite der Zentrifugationstrommel angebracht sind, und
eine Vielzahl kleiner Magnete, die in den Reaktoren platziert werden, um den Inhalt der Reaktoren bei Rotorbewegungen zu rühren.

9. Der Apparat zur parallelen Oligomersynthese nach einem der vorhergehenden Ansprüche, wobei die Dosiervorrichtung eine programmierbare Spritzenpumpe ist.

10. Der Apparat zur parallelen Oligomersynthese nach einem der vorhergehenden Ansprüche, ferner umfassend:
eine Zentrifugationstrommel zum Platzieren der Zentrifuge, und
einen Kasten, wobei der Kasten eine kreisförmige Öffnung zum Platzieren der Zentrifugationstrommel und des Abflussrohrensystems aufweist.

11. Der Apparat zur parallelen Oligomersynthese nach Anspruch 10, ferner umfassend:
eine versiegelte Abdeckung, positioniert über der Zentrifugationstrommel, wobei die versiegelte Abdeckung einen ersten Teil und einen zweiten Teil umfasst, der erste Teil besteht aus Glas oder Plexiglas und der zweite Teil besteht aus demselben inerten Material wie die Zentrifuge.

12. Ein Verfahren zur parallelen Peptidsynthese auf Feststoffbasis, das in dem Apparat zur parallelen Oligomersynthese gemäß einem der Ansprüche 1 bis 11 durchgeführt wird und dem Zeitprotokoll des Apparats folgt, **dadurch gekennzeichnet, dass** es die folgenden Schritte umfasst:
a) Harzkügelchen, die mit funktionellen Gruppen versehen sind, die zur Immobilisierung von Aminosäuren geeignet sind, werden in den Reaktor gegeben; optional ausgestattet mit einem Magneten im Reaktor;
b) die Kontrollvorrichtung ruft die Sequenz des zu synthetisierenden Peptids ab und bestimmt das sequenzspezifische Zeitprotokoll;
c) die Dosiervorrichtung gibt die gemessene Menge der ersten N-geschützten Aminosäure durch die Hebe- und Positionierungsvorrichtung an die erste der voraktivierenden Kammern des Verteilungsrotors ab;
d) der Verteilungsrotor dreht sich, um der zweiten Voraktivierungskammer einen Empfang des Inhalts der Dosiervorrichtung durch eine Hebe- und Positionierungsvorrichtung zu ermöglichen;
e) Schritte c) und d) höchstens wiederholen, bis jede Voraktivierungskammer N-geschützte Aminosäurelösung gemäß dem Zeitsteuerungsprotokoll enthält;
f) die Dosiervorrichtung gibt die gemessene Menge einer Lösung einer Carboxylgruppenaktivierungsverbindung durch die Hebe- und Positionierungsvorrichtung an die erste der voraktivierenden Kammern des Verteilungsrotors ab;
g) der Verteilungsrotor dreht sich, um der zweiten Voraktivierungskammer einen Empfang des Inhalts der Dosiervorrichtung durch eine Hebe- und Positionierungsvorrichtung zu ermöglichen;
h) Schritte f) und g) höchstens wiederholen, bis jede Voraktivierungskammer eine Lösung der Carboxylgruppenaktivierungsverbindung gemäß dem Zeitsteuerungsprotokoll enthält; ferner kann in diesem Schritt ein Farbindikator hinzugefügt werden, um den Abschluss des Kondensationsschritts 1) anzuzeigen;
i) der Verteilungsrotor wird in eine Position abgesenkt, in der er mit Reaktoren in der Zentrifuge in Kontakt kommt;
j) der Verteilungsrotor dreht sich synchron mit der Zentrifuge, wodurch der Inhalt der Voraktivierungskammern durch eine Zentrifugalkraft über Rillen in die Reaktoren übertragen wird;
k) der Verteilungsrotor löst sich durch einen Hebemechanismus von den Reaktoren und der Reaktorinhalt wird gerührt;
l) die Kondensationsreaktion von aktivierten N-geschützten Aminosäuren mit funktionellen Gruppen, die zur Immobilisierung von Aminosäuren auf den Harzkügelchen geeignet sind, verläuft für eine vorbestimmte Zeit oder alternativ, wenn ein Farbindikator hinzugefügt wurde, bis die Farbänderung eine vollständige Reaktion anzeigt.
m) die Zentrifuge mit Reaktoren dreht sich so, dass die Flüssigkeit aus den Reaktoren durch eine Zentrifugalkraft über die S-Trap-Abflüsse aus den Reaktoren übertragen wird;
n) die Dosiervorrichtung gibt die gemessene Menge eines Lösungsmittels durch die Hebe- und Positioniervorrichtung an jeden der Reaktoren der Zentrifuge ab, und die Zentrifuge mit Reaktoren dreht sich so, dass die Flüssigkeit aus den Reaktoren durch eine Zentrifugalkraft über die S-Trap-Abflüsse aus den Reaktoren übertragen wird;
o) die Dosiervorrichtung gibt die gemessene Menge eines Lösungsmittels zur Entfernung von Schutzgruppen durch die Hebe- und Positionierungsvorrichtung an jeden der Reaktoren der Zentrifuge ab, wo eine Entfernung des N-geschützten Endes der Aminosäuren stattfindet;
p) die Zentrifuge mit Reaktoren dreht sich so, dass die Flüssigkeit aus den Reaktoren durch eine Zentrifugalkraft über die S-Trap-Abflüsse aus den Reaktoren übertragen wird;
q) Wiederholen von Schritt m), um die Harzkügelchen von nicht umgesetzten Reagenzien zu waschen; ferner kann in diesem Schritt ein Farbänderungsindikator hinzugefügt werden, um den Abschluss des Kondensationsschritts 1) anzuzeigen;
r) Wiederholen der Schritte c) bis p) für zweite und folgende N-geschützte Aminosäuren gemäß dem Zeitsteuerungsprotokoll, bis die gewünschte Peptidsequenz abgeschlossen ist;
s) Abspalten des endgültigen Peptids von den Harzkügelchen durch ein Spaltmittel.

13. Das Verfahren nach Patentanspruch 12, wobei in dem Schritt k) der Reaktorinhalt durch wiederholte Hin- und Herbewegung des Rotors oder durch langsame Drehung unter Zusammenbau von Magneten gerührt wird, wenn kleine Magnete in jedem Reaktor angeordnet sind.

14. Das Verfahren nach Patentanspruch 12 oder 13, wobei der Schritt n) wiederholt wird, vorzugsweise mindestens zweimal wiederholt wird, besonders vorzugsweise wird Schritt n) fünfmal wiederholt.

15. Die Verwendung des Apparates zur parallelen Oligomersynthese nach einem der Patentansprüche 1 bis 11 und/oder des Verfahrens nach einem der Patentansprüche 12 bis 14 bei der Peptidsynthese.

## Revendications

1. Un dispositif de synthèse parallèle d'oligomères, le dispositif comprenant:
une centrifugeuse (301), la centrifugeuse comprenant
une pluralité de supports (106) de réacteur, les supports de réacteur étant configurés pour retenir les réacteurs à un angle, et
une pluralité de supports d'écoulement S-siphonner,
une pluralité de réacteurs (101), les réacteurs ayant des extrémités supérieure et inférieure, les extrémités inférieures comprenant des buses, les réacteurs étant positionnés à un angle dans les supports de réacteur,
une pluralité d'écoulements (201) S-siphonner, les écoulements S-siphonner ayant des premières extrémités et des secondes extrémités, les premières extrémités des écoulements S-siphonner étant connectées aux buses des extrémités inférieures des réacteurs, et les écoulements S-siphonner étant configuré pour fonctionner sur un principe de siphonnage,
un rotor (302) de distribution, le rotor de distribution étant pourvu de compartiments (306) de pré-activation sur sa circonférence extérieure,
les compartiments de pré-activation étant pourvus de rainures dirigées vers le bord extérieur du rotor de distribution, et configurés pour permettre le transfert de contenu des compartiments de pré-activation du rotor de distribution vers les réacteurs lors de la rotation du rotor de distribution,
un dispositif (305) de levage, le dispositif de levage étant configuré pour permettre au rotor de distribution de se déplacer dans le sens vertical entre une position supérieure et une position inférieure,
un dispositif de positionnement, le dispositif de positionnement étant configuré pour permettre une rotation du rotor de distribution en position haute indépendamment de la centrifugeuse,
un moteur d'entraînement, le moteur d'entraînement, la centrifugeuse et le rotor de distribution ayant le même axe commun, le moteur d'entraînement étant configuré pour permettre une rotation synchronisée de la centrifugeuse et du rotor de distribution en position basse,
un canal (205) d'écoulement, le canal d'écoulement étant positionné sur la circonférence extérieure de la centrifugeuse, les secondes extrémités des écoulements S-siphonner étant positionnées dans ladite rainure continue du canal d'écoulement,
le canal d'écoulement comprenant en outre une pluralité d'ouvertures d'écoulement,
un système de tuyaux de sortie, les tuyaux de sortie étant connectés à des ouvertures d'écoulement du canal d'écoulement et positionnés sous la centrifugeuse,
un dispositif (501) de dosage, le dispositif de dosage comprenant
une valve (502) de distribution, la valve de distribution ayant une pluralité d'orifices, et
une pompe (504) doseuse, la pompe doseuse étant configurée pour mesurer une quantité exacte de réactifs liquides, la pompe doseuse étant reliée à la valve de distribution,
la valve de distribution étant connectée à des solutions mères de réactifs et/ou de solvants et/ou de réactifs d'activation et/ou de réactifs de couplage via une pluralité d'orifices de la valve de distribution et via des tubes, et
un dispositif de commande, le dispositif de commande étant pourvu d'un protocole de séquence temporelle et connecté audit moteur d'entraînement, audit dispositif de dosage et audit dispositifs de levage et de positionnement.

2. Le dispositif de synthèse parallèle d'oligomères selon la revendication 1, dans lequel la centrifugeuse comprend en outre:
un premier disque en matériau inerte, le premier disque étant pourvu des supports de réacteur sur sa circonférence intérieure, et d'un deuxième ensemble d'ouvertures pour les écoulements S-siphonner sur sa circonférence extérieure,
un deuxième disque en matériau inerte, le deuxième disque étant assis sur le même axe que le premier disque et positionné en dessous du premier disque, et pourvu de fentes de fixation pour la fixation des réacteurs et d'un premier ensemble d'ouvertures pour les les écoulements S-siphonner sur sa circonférence extérieure,
de telle sorte que la pluralité de réacteurs sont situés dans les supports de réacteur du premier disque et fixés dans les fentes de fixation du deuxième disque, et
une pluralité d'écoulements S-siphonner sont connectés à la pluralité de réacteurs à travers le premier ensemble d'ouvertures dans le deuxième disque et fixés par le deuxième ensemble d'ouvertures dans le premier disque.

3. Le dispositif de synthèse parallèle d'oligomères selon l'une quelconque des revendications précédentes comprenant en outre un dispositif sélecteur, et
une pluralité de récipients de stockage pour stocker des réactifs, dans lequel le dispositif de sélection est connecté à la pluralité de récipients de stockage et au dispositif de dosage via des tubes.

4. Le dispositif selon la revendication 3, dans lequel le dispositif sélecteur comprend:
des couches coplanaires de graphite pressées ensemble par un ressort,
une pluralité d'orifices d'admission reliant le dispositif de sélection à la pluralité de conteneurs de stockage, et
un port de sortie reliant le dispositif de sélection au dispositif de dosage.

5. Le dispositif de synthèse parallèle d'oligomères selon les revendications 3 ou 4 comprenant en outre un dispositif de distribution, le dispositif de distribution étant relié par tubulure au dispositif de sélection, au dispositif de dosage et au rotor de distribution, le dispositif de distribution étant de préférence une électrovanne reliant le dispositif de dosage soit au dispositif de sélection, soit au rotor de distribution.

6. Le dispositif de synthèse parallèle d'oligomères selon l'une quelconque des revendications précédentes, dans lequel les réacteurs sont des seringues, de préférence en plastique, et dans lequel les seringues comprennent des filtres, les filtres étant de préférence faits de verre fritté, maille en plastique ou en métal, ou tout autre matériau poreux.

7. Le dispositif de synthèse parallèle d'oligomères selon l'une quelconque des revendications précédentes, dans lequel il comprend en outre un dispositif de contrôle de température, le dispositif de contrôle de température comprenant un radiateur infrarouge, un radiateur micro-ondes et un capteur de température avec contrôle par rétroaction.

8. Le dispositif de synthèse parallèle d'oligomères selon l'une quelconque des revendications précédentes, comprenant en outre
une pluralité d'aimants, la pluralité d'aimants étant fixés sur le côté intérieur du tambour de centrifugation, et
une pluralité de petits aimants à placer à l'intérieur des réacteurs, afin d'agiter le contenu des réacteurs lors des mouvements du rotor.

9. Le dispositif de synthèse parallèle d'oligomères selon l'une quelconque des revendications précédentes, dans lequel le dispositif de dosage est une pompe à seringue programmable.

10. Le dispositif de synthèse parallèle d'oligomères selon l'une quelconque des revendications précédentes, comprenant en outre
un tambour de centrifugation pour placer la centrifugeuse, et
une boîte, la boîte ayant une ouverture circulaire pour placer le tambour de centrifugation et le système de tuyaux de sortie.

11. Le dispositif de synthèse parallèle d'oligomères selon la revendication 10, comprenant en outre:
un couvercle étanche positionné sur le tambour de centrifugation, le couvercle étanche ayant une première partie et une seconde partie, la première partie en verre ou en plexiglas et la seconde partie en le même matériau inerte que la centrifugeuse.

12. Un procédé de synthèse parallèle de peptides à base solide, en utilisant le dispositif de synthèse parallèle d'oligomères selon l'une quelconque des revendications 1 à 11, et suivant le protocole de séquence temporelle du dispositif, **caractérisé en ce qu'**il comprend les étapes suivantes:
a) des billes de résine dotées de groupes fonctionnels appropriés pour l'immobilisation des acides aminés sont placées dans le réacteur; éventuellement équipé d'un aimant placé à l'intérieur du réacteur;
b) le dispositif de contrôle récupère la séquence du peptide à synthétiser et détermine le protocole de séquence temporelle spécifique à la séquence;
c) le dispositif de dosage distribue la quantité mesurée du premier acide aminé N-protégée à travers le dispositif de levage et de positionnement dans le premier des compartiments de pré-activation du rotor de distribution;
d) le rotor de distribution tourne pour permettre au deuxième compartiment de pré-activation une réception du contenu du dispositif de dosage à travers le dispositif de levage et de positionnement;
e) les étapes c) et d) se répètent au maximum jusqu'à ce que chaque compartiment de pré-activation contienne une solution d'acides aminés N-protégée selon le protocole de séquence temporelle;
f) le dispositif de dosage distribue la quantité mesurée d'une solution de composé activant le groupe carboxyle à travers le dispositif de levage et de positionnement dans le premier des compartiments de pré-activation du rotor de distribution;
g) le rotor de distribution tourne pour permettre au deuxième compartiment de pré-activation une réception du contenu du dispositif de dosage à travers le dispositif de levage et de positionnement;
h) les étapes f) et g) se répètent au maximum jusqu'à ce que chaque compartiment de pré-activation reçoive une solution de composé activant le groupe carboxyle selon le protocole de séquence temporelle; en outre, un indicateur de changement de couleur peut être ajouté dans cette étape pour indiquer la fin de l'étape de condensation 1);
i) le rotor de distribution est abaissé à une position dans laquelle il entre en contact avec les réacteurs de la centrifugeuse;
j) le rotor de distribution tourne en rotation synchronisée avec la centrifugeuse, provoquant le transfert du contenu des compartiments de pré-activation par une force centrifuge via des rainures dans les réacteurs;
k) le rotor de distribution se détache des réacteurs en soulevant le mécanisme et le contenu des réacteurs est agité;
l) la réaction de condensation des acides aminés N-protégés activés avec des groupes fonctionnels appropriés pour l'immobilisation des acides aminés sur les billes de résine se déroule pendant un temps prédéterminé, ou, en variante, si un indicateur de changement de couleur a été ajouté, jusqu'à ce que le changement de couleur indique une réaction complète;
m) la centrifugeuse avec réacteurs tourne de telle sorte que le liquide des réacteurs est transféré par une force centrifuge via les écoulements S-siphonner hors des réacteurs;
n) le dispositif de dosage distribue la quantité mesurée d'un solvant à travers le dispositif de levage et de positionnement à chacun des réacteurs de la centrifugeuse, et la centrifugeuse avec réacteurs tourne de sorte que le liquide des réacteurs est transféré par une force centrifuge via les écoulements S-siphonner hors des réacteurs;
o) le dispositif de dosage distribue la quantité mesurée d'une solution d'agent de déprotection à travers le dispositif de levage et de positionnement à chacun des réacteurs de la centrifugeuse, où une déprotection de l'extrémité N-protégée des acides aminés a lieu;
p) la centrifugeuse avec réacteurs tourne de sorte que le liquide des réacteurs est transféré par une force centrifuge via les écoulements S-siphonner hors des réacteurs;
q) l'étape m) se répète pour laver les billes de résine des réactifs n'ayant pas réagi; en outre, un indicateur de changement de couleur peut être ajouté dans cette étape pour indiquer la fin de l'étape de condensation 1);
r) les étapes c) à p) se répètent pour les deuxième et suivant acides aminés N-protégées selon le protocole de séquence temporelle, jusqu'à ce que la séquence du peptide souhaitée soit terminée;
s) clivage du peptide final des billes de résine par un agent de clivage.

13. Le procédé selon la revendication 12, dans lequel, dans l'étape k), le contenu des réacteurs est agité par des mouvements répétitifs d'avant en arrière du rotor ou par une rotation lente sous assemblage d'aimants lorsque de petits aimants sont placés dans chaque réacteur.

14. Le procédé selon la revendication 12 ou 13, dans lequel l'étape n) est répétée, de préférence elle est répétée au moins deux fois, mieux encore l'étape n) est répétée 5 fois.

15. Utilisation du dispositif de synthèse parallèle d'oligomères selon l'une quelconque des revendications 1 à 11 en synthèse peptidique.


### SEQUENCE LISTING

<110> The Institute of Organic Chemistry and Biochemistry AS CR Spyder Institute Praha, s.r.o.
<120> Device for parallel oligomer synthesis, method of parallel oligomer synthesis and use thereof
<130> XXX
<160> 25
<170> PatentIn version 3.5
<210> 1
   <211> 5
   <212> PRT
   <213> artificial
<220>
   <223> artificial peptide synthesis
<400> 1
   Tyr Gly Gly Phe Leu
   1 5
<210> 2
   <211> 5
   <212> PRT
   <213> artificial
<220>
   <223> artificial peptide synthesis
<400> 2
   Gly Gly Tyr Phe Leu
   1 5
<210> 3
   <211> 5
   <212> PRT
   <213> artificial
<220>
   <223> artificial peptide synthesis
<400> 3
   Gly Gly Tyr Leu Phe
   1 5
<210> 4
   <211> 5
   <212> PRT
   <213> artificial
<220>
   <223> artificial peptide synthesis
<400> 4
   Gly Gly Phe Tyr Leu
   1 5
<210> 5
   <211> 5
   <212> PRT
   <213> artificial
<220>
   <223> artificial peptide synthesis
<400> 5
   Gly Gly Phe Leu Tyr
   1 5
<210> 6
   <211> 5
   <212> PRT
   <213> artificial
<220>
   <223> artificial peptide synthesis
<400> 6
   Gly Gly Leu Tyr Phe
   1 5
<210> 7
   <211> 5
   <212> PRT
   <213> artificial
<220>
   <223> artificial peptide synthesis
<400> 7
   Gly Gly Leu Phe Tyr
   1 5
<210> 8
   <211> 5
   <212> PRT
   <213> artificial
<220>
   <223> artificial peptide synthesis
<400> 8
   Gly Tyr Gly Phe Leu
   1 5
<210> 9
   <211> 5
   <212> PRT
   <213> artificial
<220>
   <223> artificial peptide synthesis
<400> 9
   Gly Tyr Gly Leu Phe
   1 5
<210> 10
   <211> 5
   <212> PRT
   <213> artificial
<220>
   <223> artificial peptide synthesis
<400> 10
   Gly Tyr Phe Gly Leu
   1 5
<210> 11
   <211> 5
   <212> PRT
   <213> artificial
<220>
   <223> artificial peptide synthesis
<400> 11
   Gly Tyr Phe Leu Gly
   1 5
<210> 12
   <211> 5
   <212> PRT
   <213> artificial
<220>
   <223> artificial peptide synthesis
<400> 12
   Gly Tyr Leu Gly Phe
   1 5
<210> 13
   <211> 5
   <212> PRT
   <213> artificial
<220>
   <223> artificial peptide synthesis
<400> 13
   Gly Tyr Leu Phe Gly
   1 5
<210> 14
   <211> 5
   <212> PRT
   <213> artificial
<220>
   <223> artificial peptide synthesis
<400> 14
   Gly Phe Gly Tyr Leu
   1 5
<210> 15
   <211> 5
   <212> PRT
   <213> artificial
<220>
   <223> artificial peptide synthesis
<400> 15
   Gly Phe Gly Leu Tyr
   1 5
<210> 16
   <211> 5
   <212> PRT
   <213> artificial
<220>
   <223> artificial peptide synthesis
<400> 16
   Gly Phe Tyr Gly Leu
   1 5
<210> 17
   <211> 5
   <212> PRT
   <213> artificial
<220>
   <223> artificial peptide synthesis
<400> 17
   Gly Phe Tyr Leu Gly
   1 5
<210> 18
   <211> 5
   <212> PRT
   <213> artificial
<220>
   <223> artificial peptide synthesis
<400> 18
   Gly Phe Leu Gly Tyr
   1 5
<210> 19
   <211> 5
   <212> PRT
   <213> artificial
<220>
   <223> artificial peptide synthesis
<400> 19
   Gly Phe Leu Tyr Gly
   1 5
<210> 20
   <211> 5
   <212> PRT
   <213> artificial
<220>
   <223> artificial peptide synthesis
<400> 20
   Gly Leu Gly Tyr Phe
   1 5
<210> 21
   <211> 5
   <212> PRT
   <213> artificial
<220>
   <223> artificial peptide synthesis
<400> 21
   Gly Leu Gly Phe Tyr
   1 5
<210> 22
   <211> 5
   <212> PRT
   <213> artificial
<220>
   <223> artificial peptide synthesis
<400> 22
   Gly Leu Tyr Gly Phe
   1 5
<210> 23
   <211> 5
   <212> PRT
   <213> artificial
<220>
   <223> artificial peptide synthesis
<400> 23
   Gly Leu Tyr Phe Gly
   1 5
<210> 24
   <211> 5
   <212> PRT
   <213> artificial
<220>
   <223> artificial peptide synthesis
<400> 24
   Tyr Gly Gly Phe Leu
   1               5
<210> 25
   <211> 24
   <212> PRT
   <213> artificial
<220>
   <223> artificial peptide synthesis
<220>
   <221> MISC_FEATURE
   <222> (7)..(7)
   <223> SSO3H
<220>
   <221> MISC_FEATURE
   <222> (13)..(13)
   <223> SSO3H
<220>
   <221> MISC_FEATURE
   <222> (23)..(23)
   <223> SSO3H
<400> 25
   Gly Ile Val Glu Gln Cys Xaa Cys Thr Ser Ile Cys Xaa Ser Leu Tyr
   1               5                   10                  15
   Gln Leu Glu Asn Tyr Cys Xaa Asn
               20